# EUROPEAN PATENT SPECIFICATION

(11) **EP 4 188 420 B1**
(45) Date of publication and mention of the grant of the patent: **05.06.2024**
(21) Application number: 22712997.0
(22) Date of filing: 16.02.2022
(51) Int. Cl.: A61K 38/21, A61P 31/14, C07K 14/56

(54) **COMPOSITIONS AND METHODS RELATING TO THE TREATMENT OF DISEASES**
ZUSAMMENSETZUNGEN UND VERFAHREN IM ZUSAMMENHANG MIT DER BEHANDLUNG VON KRANKHEITEN
COMPOSITIONS ET MÉTHODES SE RAPPORTANT AU TRAITEMENT DE MALADIES

(30) Priority: 17.02.2021 GB 202102261
(43) Date of publication of application: 07.06.2023
(73) Proprietor: ILC Therapeutics Limited, Newhouse, Lanarkshire ML1 5UH (GB)
(72) Inventor: STIMSON, William, Newhouse Lanarkshire ML1 5UH (GB)
(74) Representative: Murgitroyd & Company
(86) International application number: PCT/GB2022/050406
(87) International publication number: WO 2022/175651

(56) References cited:
- WO-A1-2019/229480
- WO-A1-2021/186162
- WO-A2-2006/111745
- WO-A2-2015/136287
- JEFFY GEORGE ET AL: "Interferon-? Subtypes As an Adjunct Therapeutic Approach for Human Immunodeficiency Virus Functional Cure", FRONTIERS IN IMMUNOLOGY, vol. 9, 22 February 2018 (2018-02-22), XP055769094, DOI: 10.3389/fimmu.2018.00299
- LI GUANGDI ET AL: "Therapeutic options for the 2019 novel coronavirus (2019-nCoV)", NATURE REVIEWS DRUG DISCOVERY, NATURE PUBLISHING GROUP, GB, vol. 19, no. 3, 10 February 2020 (2020-02-10), pages 149-150, XP037050085, ISSN: 1474-1776, DOI: 10.1038/D41573-020-00016-0 [retrieved on 2020-02-10]

## Description

### Field of the invention

The present invention relates to compositions for preventing or treating a viral infection. The invention extends to compositions for preventing or treating infection from viral infections by inducing NK and T cell responsiveness. The invention further extends to compositions and the use of the compositions of the invention for the treatment and/or prophylaxis of a viral infection for Human and Veterinary therapies.

### Background to the Invention

Interferon-alpha (IFN-alpha) has been used clinically and commercially (e.g., RoferonA^{®}, IntronA^{®}, Pegasys^{®}, Pegintron^{®} etc) to successfully treat various viruses such as severe acute respiratory syndrome (SARS), chronic Hepatitis B, chronic Hepatitis C and HIV. IFN-alpha is one of the earliest cytokines released by antigen presenting cells as part of the innate immune response. It is directly responsible for NK and T cell responsiveness.

It is known that different pathogens induce different Interferon-alpha (IFN-α) subtypes *in vitro* and that IFN-α subtypes have different anti-viral, anti-proliferative and immunomodulatory activities. The mechanisms of actions of IFN-α, and in particular individual IFN-α subtypes, are still only partly understood. Infection via a variety of routes has been shown to induce different subtype profiles. IFN-α subtypes bind to the same receptors, activate common signaling pathways and had been expected to have similar immunological functions. All IFN-α subtypes have anti-viral activities, by definition, although their absolute efficacy in this context may vary considerably. In addition, many other biological properties have been described, but with varying potencies, including immunomodulatory and anti-proliferative activities. The pleiotropic effects appear to be due to differential interaction with the receptor chains and signaling through different intracellular pathways to an array of effector molecules. The Type I IFN receptor consists of two chains, IFNR1 and IFNR2. There is a range of binding affinities for each of the 12 IFN-α subtypes with the different receptor chains. IFNα-14 has one of the highest affinities for both of the two interferon receptors, which is why it is so active compared to the other 11 subtypes.

Recombinant interferons, which consist of only the IFN alpha 2 subtype, currently dominate the market for anti-viral indications. There are two main recombinant alpha IFN products, Intron A^{™} from Schering Plough (IFN-alpha 2b) and Roferon^{™} (IFN-alpha 2a) from Roche. In contrast to these single-subtype products, there are several alpha IFN preparations that consist of a mixture of different subtypes. These multi-subtype IFN alpha products are produced either by human leukocytes in response to a stimulation from a virus (such as Multiferon^{™} from Viragen, Inc or its subsidiaries, or Alferon-N^{™} from Interferon Sciences/Hemispherix), or in human lymphoblastoid cells, cultured from a patient with Burkitt's lymphoma (such as Sumiferon^{™} from Sumitomo).

### Summary of Invention

The present invention is as defined in the claims.

In the absence of vaccines targeted against specific viruses, particularly newly emerging viruses, an effective antiviral drug would be particularly advantageous, suitably in relation to outbreaks of SARs, influenza (particularly H5N1), Zika virus, WNV and EBOV.

Accordingly, a first aspect of the present invention provides a composition for the treatment and/or prophylaxis of viral infection, said method comprising the step of:
(i) administering to a subject in need thereof a therapeutically effective amount of HYBRID 2.

Following extensive experimentation, the inventor of the present invention has surprisingly discovered that administering, a variant of interferon alpha 14 HYBRID 2, SEQ ID NO: 3 or a variant or fragment thereof as described herein results in the suppression or inhibition of the effects of viral infection. The inventor unexpectedly determined that HYBRID 2 can directly inhibit the cytopathic effect resulting from viral infection. The cytopathic effect or cytopathogenic effect refers to structural changes in host cells that are caused by viral invasion. The inventor demonstrated that HYBRID 2 also inhibit plaque formation relating to viral infection.

The present inventor has examined the ability of the synthetic alpha-interferon Hybrid 2 to inhibit viral infection using a model system based on the cytopathic effect and plaque formation caused as a result of infection with SARS-CoV-1 and SARS-CoV-2. The inventor has also examined the inhibition of the cytopathic effect in comparison with the anti-viral compound Ribavirin and the multi-subtype interferon Multiferon^{™}.

Additionally the inventor has utilised a HIV and a RSV model to demonstrate the anti-viral effect of HYBRID 2. The inventor utilized different adherent cell lines to demonstrate the antiviral activity of HYBRID 2 against HIV and HRSV. For each antiviral assay, a viability test was set up in parallel using the same concentrations of inhibitors tested in the antiviral assays. Viability assays were used to determine compound-induced cytotoxicity effects in the absence of virus. There are over 37 million people worldwide with HIV. RSV infection is the most important cause of hospitalisation in infants and one of the leading causes of infant mortality. As one of the respiratory viruses, along with influenza, rhioviruses and coronaviruses, it is both interesting in its own right and provides an indicative model. The inventor has also demonstrated that treatment of primary human keratinocytes with HYBRID 2 reduces Herpes Simplex Virus Type 1 (HSV1) infection.

Suitably a viral infection may be treated by providing HYBRID 2 (SEQ ID NO:3) or a variant or fragment thereof, by direct injection or where appropriate, for example for viruses that cause respiratory issues by aerosol directly into the lungs. Suitably doses in the range 10⁴ to 10⁷ IU per administration may be used. Suitably, HYBRID 2 (SEQ ID NO:3) may be provided as a sublingual treatment. Administration of HYBRID 2 or a variant or fragment thereof as a sublingual treatment may result in a greater reduction or inhibition of viral activity compared to previous anti-viral medications. In addition, the inventors have determined that very low doses of HYBRID 2 for example up to 10⁴ to 5×10⁶ IU per day may be used.

This has led to the identification by the inventor of improved therapeutic compositions which have utility in the treatment and/or prophylaxis of viral infection.

Suitably the viral infection may be selected from virus selected from a member of the Flaviviridae family (e.g., a member of the Flavivirus, Pestivirus, and Hepacivints genera), which includes the hepatitis C virus, Yellow fever virus; Tick-borne viruses, such as the Gadgets Gully virus, Kadam virus, Kyasanur Forest disease virus, Langat virus, Omsk hemorrhagic fever virus, Powassan virus, Royal Farn1 virus, Karshi virus, tick-borne encephalitis virus, Neudoerfl virus, Sofjin virus, Louping ill virus and the Negishi virus; seabird tick-borne viruses, such as the Meaban virus, Saumarez Reef virus, and the Tyuleniy virus; mosquito-borne viruses, such as the Arna virus, dengue virus, Kedougou virus, Cacipacore virus, Koutango virus, Japanese encephalitis virus, Murray Valley encephalitis virus, St. Louis encephalitis virus, Usutu virus, West Nile virus, Yammde virus, Kokobera virus, Bagaza virus, Ilheus virus, Israel turkey meningoencephalo-myelitis virus, Ntaya virus, Tembusu virus, Zika virus, Barizi virus, Bouboui vims, Edge Hill virus, Jugra virus, Saboya virus, Sepik virus, Uganda S virus, Wesselsbron virus, yellow fever virus; Entebbe bat virus, Yokose virus, Apoi virus, Cowbone Ridge virus, Jutiapa virus, Modoc virus, Sal Vieja virus, San Perlita virus, Bukalasa bat virus, Carey Island virus, Dakar bat virus, Montana myotis leukoencephalitis virus, Phnom Penh bat virus, Rio Bravo virus, Tamana bat virus, and the Cell fusing agent virus.

In another embodiment, the virus is selected from a member of the Arenaviridae family, which includes the Ippy virus, Lassa virus (e.g., the Josiah, LP, or GA391 strain), lymphocytic choriomeningitis virus (LCMV), Mobala virus. Mopeia virus, Amapari virus, Flexal virus, Guanarito virus, Junin virus, Latino virus, Machupo virus, Oliveros virus, Parana virus, Pichinde virus, Pirital virus, Sabia virus, Tacaribe virus, Tamiami virus, Whitewater Arroyo virus, Chapare virus, and Lujo virus.

In yet other embodiments the virus can be selected from a member of the Bunyaviridae family (e.g., a member of the *Hantavirus, Nairovirus, Orthobunyavirus,* and *Phlebovirus* genera), which includes the Hantaan virus, Sin Nombre virus, Dugbe virus, Bunyamwera virus, Rift Valley fever virus, La Crosse virus, Punta Toro virus (PTV), California encephalitis virus, and Crimean-Congo hemorrhagic fever (CCHF) virus, a virus from the Filoviridae family, which includes the Ebola virus (e.g., the Zaire, Sudan, Ivory Coast, Reston, and Uganda strains) and the Marburg virus (e.g., the Angola, Ci67, Musoke, Popp, Ravn and Lake Victoria strains);

In embodiments the virus can be selected from a member of the Togaviridae family (e.g., a member of the *Alphavirus* genus), which includes the Venezuelan equine encephalitis virus (VEE), Eastern equine encephalitis virus (EEE), Western equine encephalitis virus (WEE), Sindbis virus, rubella virus, Semliki Forest virus, Ross River virus, Barmah Forest virus, O' nyong'nyong virus, and the chiklmgunya virus; a member of the Poxyiridae family (e.g., a member of the *Orthopoxvirus* genus), which includes the smallpox virus, cowpox, moukeypox virus, and vaccinia virus; a member of the Herpesviridae family, which includes the herpes simplex virus (HSV; types 1, 2, and 6), human herpes virus (e.g., types 7 and 8), cytomegalovirus (CMV), Epstein-Barr virus (EBV), Varicella-Zoster virus, and Kaposi's sarcoma associated-herpesvirus (KSHV).

In another embodiment the virus can be selected from a member of the Orthomyxoviridae family, which includes the influenza virus (A, B, and C), such as the H5Nl avian influenza virus or HlNl swine flu; a member of the Rhabdoviridae family, which includes the rabies virus and vesicular stomatitis virus (VSV); a member of the Paramyxoviridae family, which includes the human respiratory syncytial virus (RSV), Newcastle disease virus, hendravirus, nipahvirus, measles virus, rinderpest virus, canine distemper virus, Sendai virus, human parainfluenza virus (e.g., 1, 2, 3, and 4), rhinovirus, and mumps virus.

In embodiments the virus can be selected from a member of the Picornaviridae family, which includes the poliovirus, human enterovirus (A, B, C, and D), hepatitis A virus, and the coxsackievirus; a member of the Hepadnaviridae family, which includes the hepatitis B virus.

In embodiments the virus can be selected from a member of the Retroviridae family, which includes the human immunodeficiency vims (HIV; e.g., types 1 and 2), and human T-lymphotropic vims Types I and II (HTLV-1 and HTLV-2, respectively).

In embodiments the virus can be selected from echo virus Suitably the virus may be selected from a virus which encodes a protein known to antagonise the type 1 IFN response:
Chikungunya virus (CHIKV)
Coxsackievirus
Dengue virus (DENV)
Epstein-Barr virus (EBV
Hepatitis B virus (HBV)
Hepatitis C virus (HCV)
Human cytomegalovirus (HCMV)
Human immuno-deficiency virus (HIV)
Human parainfluenza virus (HPIV)
Human respiratory syncytial virus (HRSV)
Human rhinovirus (HRV)
Herpes simplex virus (HSV)
Influenza A virus (IAV)
Influenza B virus (IBV)
Lassa virus (LASV)
Measles virus (MeV)
Mumps virus (MuV)
Nipah virus (NiV)
Poliovirus (PV)
Rabies virus (RABV)
West Nile virus (WNV)
Yellow fever virus (YFV)
Zika Virus (ZIKV).

Suitably the virus may be selected from a viral disease caused by an enveloped virus, preferably selected from the group (not all claimed) consisting of: Human Immunodeficiency Virus 1 (HIV-l),
Human Immunodeficiency Virus 2 (HIV-2), Herpes 1 virus (HSV-l), Herpes 2 virus (HSV-2), Influenza Virus, Respiratory Syncytial Virus (RSV), Cytomegalovirus (CMV), Zika Virus (ZKV), Dengue Virus, West Nile Virus, Lassa Virus, Ebola Virus, Lloviu virus, Bundibugyo virus, Reston virus, Sudan virus, Tai Forest virus, Marburg virus, Ravn virus (RAW), Pneumovirus, Junin Virus, Rift Valley fever virus, La Crosse Virus, Porcine Reproductive And Respiratory Syndrome Virus, Poxvirus, Bovine Viral diarrhoea Norovirus, SARS Coronavirus, Chikunguya Virus, Hepatitis C Virus, Hepatitis B Virus, Schmallenberg virus, African swine fever virus, Eastern Equine Encephalitis Virus, Cowpox virus, Western Equine Encephalitis Virus, Nipah Virus, Omsk hemorrhagic fever Virus, Venezuelan Equine Encephalitis Virus, Human parainfluenza viruses, Japanese Encephalitis Virus, Tick Borne Encephalitis Virus, Russian spring-summer encephalitis (RSSE) virus, Yellow Fever Virus, Newcastle Virus, Virus (BVDV), Parainfluenza virus type 5 (PIV5), Border Disease Virus (BDV) of sheep, Classical Swine Fever Virus (CSFV), Vesicular Stomatitis Virus (VSV) and HSV-2 acyclovir resistant (HSV-2 Acy R), more preferably the enveloped virus is selected from the group consisting of: HIV-l, HSV-l, HSV-2, HCMV, RSV, VSV, H1N1, DENV-2 and ZKV.

In embodiments, the viral infection may be selected from Human Immunodeficiency Virus (HIV), Human Respiratory Syncytial Virus (HRSV) or Herpes Simplex Virus (HSV).

The interferon alpha subtype HYBRID 2 comprises or consists of an amino acid sequence SEQ ID NO:3 or a functionally active fragment or variant thereof.

In embodiments, the method of administration is oral administration. In embodiments, the method of administration is injection. In embodiments the method of administration is by aerosol delivery to the lungs.

In embodiments, the therapeutically effective amount of the interferon alpha subtype HYBRID 2 is a low dose. In embodiments, the therapeutically effective amount of the interferon alpha subtype HYBRID 2 is between 10⁴ to 5×10⁶ IU units/ml. In embodiments, the therapeutically effective amount of the interferon alpha subtypeHYBRID 2 is lower than current systemic treatments for coronavirus infection.

In embodiments, the interferon alpha subtype HYBRID 2 is administered in a dose of 5IU/ml, 10IU/ml, 50IU/ml, 1×10²IU/ml, 1×10³IU/ml, 1×10⁴IU/ml, 1×10⁵IU/ml, 1×10⁶IU/ml or 1×10⁷IU/ml.

In embodiments, the interferon alpha subtype HYBRID 2 is administered in a dose of between 0.1mg to 1mg, suitably 1ng -50 micrograms. For example, in human applications 5×10⁴ IU/ml units or less may be used.

In embodiments, the interferon alpha subtype HYBRID 2 is administered by sublingual administration. In embodiments, the interferon alpha subtype can be administered once a day, twice a day, three times a day or four times a day. Suitably, in sublingual administration, a single dose may be provided each day.

In embodiments, the interferon alpha subtype HYBRID 2 interrupts the viral activity, for example inhibits the cytopathic effect or the plaque formation of the virus.

Typically, the subject is a mammal, in particular a human. In embodiments the subject can be a bird. In embodiments the subject can be an animal.

In certain embodiments, the method includes the step of administering a therapeutically useful amount of a suitable anti-viral compound. In embodiments, the anti-viral compound is ribavirin. In embodiments in combination with the interferon alpha subtype, HYBRID 2 a further treatment may be selected from Remdesivir, LAM-002A (apilimod - a selective PIKfyve kinase inhibitor), dexamethasone, and Avigan (favilavir) or another interferon subtype.

According to a second aspect of the present invention, there is provided an interferon alpha subtype, HYBRID 2 for use in the treatment and/or prophylaxis of a viral infection.

The interferon alpha subtype HYBRID 2 comprises or consists of an amino acid sequence SEQ ID NO:3 or a functionally active fragment or variant thereof.

In embodiments, the interferon alpha subtype, HYBRID 2 is for use in the treatment and/or prophylaxis of respiratory viral disease, gastrointestinal viral disease, exanthematous viral disease, hepatic viral disease, cutaneous viral disease, haemorrhagic viral disease, neurological viral disease.

In embodiments, the interferon alpha subtype, HYBRID 2 is for use in the treatment and/or prophylaxis of Human Immunodeficiency Virus (HIV), Human Respiratory Syncytial Virus (HRSV) and Herpes Simplex Virus.

In embodiments, the interferon alpha subtype, HYBRID 2 is for use in the treatment and/or prophylaxis of Human Immunodeficiency Virus (HIV), Human Respiratory Syncytial Virus (HRSV) or Herpes Simplex Virus (HSV).

In embodiments, the therapeutically effective amount of HYBRID 2 is a low dose. In embodiments, the therapeutically effective amount of the interferon alpha subtype HYBRID 2 is between 10⁴ to 5×10⁶ IU units/ml.

In embodiments, the HYBRID 2 is administered in a dose of 5IU/ml, 10IU/ml, 50IU/ml, 1×10²IU/ml, 1×10³IU/ml, 1×10⁴IU/ml, 1×10⁵IU/ml, 1×10⁶IU/ml or 1×10⁷IU/ml.

In embodiments, the HYBRID 2 is administered in a dose of between 0.1mg to 1mg, suitably 1ng -50 micrograms. For example, in human applications 5×10⁴ IU/ml units or less may be used. In animals, e.g. dog, sublingual use may be 10⁴ IU/Kg, for example in 1ml PBS.

In embodiments, the the interferon alpha subtype can be administered once a day, twice a day, three times a day or four times a day. Suitably, in sublingual administration, a single dose may be provided each day.

In embodiments in combination with r HYBRID 2 a further treatment may be selected from Remdesivir, LAM-002A (apilimod - a selective PIKfyve kinase inhibitor), dexamethasone, and Avigan (favilavir).

According to a third aspect of the present invention, there is provided use of HYBIRD 2 or a combination thereof in the preparation of a medicament for the treatment and/or prophylaxis of viral infection.

According to a further aspect of the present invention, there is provided a composition comprising HYBRID 2, for use in the treatment and/or prophylaxis of Human Immunodeficiency Virus (HIV), Human Respiratory Syncytial Virus (HRSV) or Herpes Simplex Virus (HSV).

According to a further aspect of the present invention, there is provided a pharmaceutical composition comprising HYBRID 2, for use in the treatment and/or prophylaxis of viral infection.

In embodiments, the viral infection may be selected from Human Immunodeficiency Virus (HIV), Human Respiratory Syncytial Virus (HRSV) or Herpes Simplex Virus (HSV).

In embodiments the pharmaceutical composition may provide HYBRID 2 in combination with a further treatment, wherein the further treatment may be selected from Remdesivir, LAM-002A (apilimod - a selective PIKfyve kinase inhibitor), dexamethasone, and Avigan (favilavir).

According to a further aspect of the present invention, there is provided HYBRID 2 for use in modulating an immune response.

In embodiments a combination of the interferon alpha subtype HYBRID 2 with a further treatment selected from Remdesivir, LAM-002A (apilimod - a selective PIKfyve kinase inhibitor), dexamethasone, and Avigan (favilavir) can be used to modulate the immune response.

According to a further aspect of the present invention there is provided the use of HYBRID 2 or a combination thereof and an anti-viral compound for the treatment or prevention of infection with a virus, and in Human Immunodeficiency Virus (HIV), Human Respiratory Syncytial Virus (HRSV) or Herpes Simplex Virus (HSV). Suitably a virus may be selected from: Chikungunya virus (CHIKV)
Coxsackievirus
Dengue virus (DENV)
Epstein-Barr virus (EBV
Hepatitis B virus (HBV)
Hepatitis C virus (HCV)
Human cytomegalovirus (HCMV)
Human immuno-deficiency virus (HIV)
Human parainfluenza virus (HPIV)
Human respiratory syncytial virus (HRSV)
Human rhinovirus (HRV)
Herpes simplex virus (HSV)
Influenza A virus (IAV)
Influenza B virus (IBV)
Lassa virus (LASV)
Measles virus (MeV)
Mumps virus (MuV)
Nipah virus (NiV)
Poliovirus (PV)
Rabies virus (RABV)
West Nile virus (WNV)
Yellow fever virus (YFV)
Zika Virus (ZIKV).

The interferon alpha subtype HYBRID 2 comprises or consists of an amino acid sequence SEQ ID NO:3 or a functionally active fragment thereof.

In embodiments of the aspects of the invention outlined above, the composition or pharmaceutical composition is administered sublingually. This may be particularly advantageous for veterinary treatments. In embodiments, the method of administration is oral administration. In embodiments, the method of administration is injection.

In embodiments of the aspects of the invention outlined above, HYBRID 2 is administered in a dose of 5IU/ml, 10IU/ml, 50IU/ml, 1×10²IU/ml, 1×10³IU/ml, 1×10⁴IU/ml, 1×10⁵IU/ml, 1×10⁶IU/ml or 1×10⁷IU/ml.

In embodiments of the aspects of the invention outlined above, HYBRID 2 is administered in a dose of between 0.1mg to 1mg, suitably 1ng to 50 micrograms. For example, in human applications 5×10⁴ IU/ml units or less may be used.

In embodiments of the aspects of the invention outlined above, the interferon alpha subtype is administered once a day, twice a day, three times a day or four times a day. Suitably, in sublingual administration, a single dose may be provided each day.

In certain embodiments of the aspects of the invention outlined above, the IFN-α subtype comprises, or consists of the amino acid sequence SEQ ID NO:3 (HYBRID 2) or a fragment thereof. In a further aspect of the invention there is provided a recombinant polypeptide comprising or consisting of SEQ ID NO:3 or a fragment thereof. The invention extends to nucleic acid sequences derived from the amino acid sequence SEQ ID NO:3. In embodiments the HYBRID 2 can be glycosylated.

### Description

There are many differences between the recombinant forms of IFN alpha in the art and the multi-subtype forms. The most obvious difference is the number of IFN alpha subtypes each possesses. The recombinant forms comprise only the alpha 2 subtype - the alpha 2b form for Intron A^{™} (Schering Plough) and the alpha 2a form for Roferon^{™} (Roche). The multi-subtype forms of IFN alpha comprise many subtypes of IFN alpha. The multi-subtype forms of IFN alpha are a broad spectrum drug and there is no indication that any one of the IFN alpha subtypes has more or less anti-viral activity. Another difference between the multi-subtype and the recombinant forms is that the IFN alpha 2 produced by human cells in the manufacturing process of the multi-subtype forms is glycosylated, whereas the recombinant forms are unglycosylated, in that they are produced through bacterial fermentation. Glycosylation plays a major role in many functions of the protein product, such as half-life, the bioactivity and its immunogenicity. Therefore, the glycosylation of a product is an important consideration when developing a therapeutic or prophylactic treatment, as it may affect the duration in the body after administration, the activity of a therapeutically appropriate dose and the tolerability to the product itself.

SEQ ID NO:3 (HYBRID 2 of the present invention) has an amino acid sequence as follows:

CDLPQTHSLGNRRALILLGQMGRISPFSCLKDRHDFRIPQEEFDGNQFQKAQAISVLHEM
MQQTFNLFSTKNSSAAWDETLLEKFYIELFQQMNDLEACVIQEVGVEETPLMNEDSILAV
KKYFQRITLYLIERKYSPCAWEVVRAEIMRSLSFSTNLQKRLRRKD

In particular, the inventor has discovered that HYBRID 2 (SEQ ID NO:3) or a variant or fragment thereof, inhibits the cytopathic effect and plaque formation and demonstrates antiviral activity. The cytopathic effect or cytopathogenic effect refers to structural changes in host cells that are caused by viral invasion.

The inventor has also established that the recombinant IFN-hybrid molecule HYBRID 2 (SEQ ID NO: 3) has a high binding affinity to the interferon receptors.

Whilst not wishing to be bound by theory, the inventor believes that proteins comprising the amino acid sequence of IFN-α10 have greater affinity to interferon receptor 2 (IFNR2) and proteins comprising the amino acid sequence of IFN-α14 have greater affinity to interferon receptor 1 (IFNR1). Thus, substitution of a protein comprising an IFN-α10 amino acid sequence with amino acids of IFN-α14 which allow binding to interferon receptor 1 or substitution of a protein comprising an IFN-α14 amino acid sequence with amino acids of IFN-α10 which allow binding to interferon receptor 2 is considered to provide a IFN-α10 IFN-α14 hybrid protein which should have stronger binding affinity to both interferon receptors 1 and 2 than IFN-α10 or IFN-α14 alone. By including the primary interferon receptor binding sites of IFN-α10 and IFN-α14 is meant that the hybrid comprises amino acids selected from IFN-α10 and substituted into an IFN-α14 amino acid sequence to improve the ability of an IFN-α14 subtype to bind to an interferon receptor 2 and / or that the hybrid comprises amino acids selected from IFN-α14 and substituted into an IFN-α10 amino acid sequence to improve the ability of an IFN-α10 subtype to bind to an interferon receptor 1. HYBRID 2 is considered to be particularly advantageous in the treatment of virus.

The IFN-α10-IFN-α14 hybrid can substantially have the amino-acid sequence of IFN-α10, but be modified in a region between amino residues 80 to 150, or suitably between amino acid residues 84 to 144, or suitably amino acid residues 92 to 115 or suitably between amino acid residues 90 to 110, (utilizing the numbering of the IFN-α10 sequence) to provide the amino acids provided by the IFN-α14 sequence. It is considered the amino acid residues in these regions or parts of these regions provide for the binding of IFN-α14 to interferon receptor 1. In particular, the hybrid sequence may include at least one, at least two, at least three, at least 4, at least 5, at least 6, at least 7, at least 8, at least 9, at least 10 or at least 11 modifications of the IFN-α10 sequence to provide the corresponding residues of the IFN-α14 sequence or a conserved mutation thereof. In embodiments, eleven modifications are provided as indicated by the amino acids noted in bold.

CDLPQTHSLGNRRALILLGQMGRISPFSCLKDRHDFRIPQEEFDGNQFQKAQAISVLHEM
MQQTFNLFSTKNSSAAWDETLLEKFYIELFQQMNDLEACVIQEVGVEETPLMNEDSILAV
KKYFQRITLYLIERKYSPCAWEVVRAEIMRSLSFSTNLQKRLRRKD
(HYBRID 2: SEQ ID NO: 3).

By functionally active is meant an IFN-α10-IFN- α14 hybrid polypeptide comprising the primary interferon binding sites of IFN-α10 and IFN-α14 wherein the administration of peptide to a subject or expression of peptide in a subject promotes suppression of an immune response to a viral infection. Further, functional activity may be indicated by the ability of a hybrid peptide to suppress an immune response to a viral infection.

A fragment can comprise at least 50, preferably 100 and more preferably 150 or greater contiguous amino acids from SEQ ID NO: 3 and which is functionally active. Suitably, a fragment may be determined using, for example, C-terminal serial deletion of cDNA. Said deletion constructs may then be cloned into suitable plasmids. The activity of these deletion mutants may then be tested for biological activity as described herein.

By variant is meant an amino acid sequence which is at least 90% homologous to SEQ ID NO: 3, more preferably at least 95% homologous to SEQ ID NO: 3, more preferably at least 97% homologous to SEQ ID NO: 3, even more preferably at least 98% homologous to SEQ ID NO: 3, even more preferably at least 99% homologous to SEQ ID NO: 3. A variant encompasses a polypeptide sequence of SEQ ID NO: 3 which includes substitution of amino acids, especially a substitution(s) which is/are known for having a high probability of not leading to any significant modification of the biological activity or configuration, or folding, of the protein. These substitutions, typically known as conserved substitutions, are known in the art. For example the group of arginine, lysine and histidine are known interchangeable basic amino acids. Suitably, in embodiments amino acids of the same charge, size or hydrophobicity may be substituted with each other. Suitably, any substitution may be selected based on analysis of amino acid sequence alignments of interferon alpha subtypes to provide amino acid substitutions to amino acids which are present in other alpha subtypes at similar or identical positions when the sequences are aligned. Hybrids, and variants and fragments thereof may be generated using suitable molecular biology methods as known in the art. Suitably homology may be described by sequence identity. Sequence identity can be determined by methods as known in the art, for example BLAST.

The inventor has discovered that administration of HYBRID 2 (SEQ ID NO:3) or a variant or fragment thereof, results in a 10%, preferably a 20%, preferably a 30%, preferably a 40%, preferably a 50%, preferably a 60%, preferably a 70%, preferably a 80% and more preferably a 90% greater reduction of viral activity, such as cytopathic effect or plaque formation or in antiviral assays, compared to controls to which HYBRID 2 (SEQ ID NO:3) or a variant or fragment thereof have not been administered.

The inventor has discovered that administration of HYBRID 2 (SEQ ID NO:3) or a variant or fragment thereof, results in a 10%, preferably a 20%, preferably a 30%, preferably a 40%, preferably a 50%, preferably a 60%, preferably a 70%, preferably a 80% and more preferably a 90% greater reduction of viral activity, such as cytopathic effect or plaque formation or in antiviral assays, compared to previous anti-viral medications.

Administration of HYBRID 2 can reduce cytopathic effect or plaque formation by 50%, preferably by 60%, preferably by 70%, preferably by 80%, preferably by 90%, preferably by 91%, preferably by 92%, preferably by 93%, preferably by 94%, preferably by 95%, preferably by 96%, preferably by 97%, and more preferably by 98%.

### Definitions

### Fragment

A fragment can comprise at least 50, preferably 100 and more preferably 150 or greater contiguous amino acids from SEQ ID NO: 1, SEQ ID NO:2 or SEQ ID NO:3 and which is functionally active. Suitably, a fragment may be determined using, for example, C-terminal serial deletion of cDNA. Said deletion constructs may then be cloned into suitable plasmids. The activity of these deletion mutants may then be tested for biological activity as described herein. Fragments may be generated using suitable molecular biology methods as known in the art.

### Variant

By variant is meant an amino acid sequence which is at least 90% homologous to SEQ ID NO:3, even more preferably at least 95% homologous to SEQ ID NO:3, even more preferably at least 96% homologous to SEQ ID NO:3, even more preferably at least 97% homologous to SEQ ID NO:3, and most preferably at least 98% homology with SEQ ID NO:3. A variant encompasses a polypeptide sequence of SEQ ID NO:3 which includes substitution of amino acids, especially a substitution(s) which is/are known for having a high probability of not leading to any significant modification of the biological activity or configuration, or folding, of the protein. These substitutions, typically known as conserved substitutions, are known in the art. For example the group of arginine, lysine and histidine are known interchangeable basic amino acids. Suitably, in embodiments amino acids of the same charge, size or hydrophobicity may be substituted with each other. Suitably, any substitution may be selected based on analysis of amino acid sequence alignments of interferon alpha subtypes to provide amino acid substitutions to amino acids which are present in other alpha subtypes at similar or identical positions when the sequences are aligned. Variants may be generated using suitable molecular biology methods as known in the art.

### Subject

As herein defined, a "subject" includes and encompasses mammals such as humans, primates and livestock animals (e.g. sheep, pigs, cattle, horses, donkeys); laboratory test animals such as mice, rabbits, rats and guinea pigs; and companion animals such as dogs and cats.

### Treatment / Therapy

The term "treatment" is used herein to refer to any regimen that can benefit a human or non-human animal. The treatment may be in respect of a viral infection and the treatment may be prophylactic (preventative treatment). Treatment may include curative or alleviative effects. Reference herein to "therapeutic" and "prophylactic" treatment is to be considered in its broadest context. The term "therapeutic" does not necessarily imply that a subject is treated until total recovery. Similarly, "prophylactic" does not necessarily mean that the subject will not eventually contract a disease condition. Accordingly, therapeutic and/or prophylactic treatment includes amelioration of the symptoms of a viral infection or preventing or otherwise reducing the risk of developing a viral infection. The term "prophylactic" may be considered as reducing the severity or the onset of a particular condition. "Therapeutic" may also reduce the severity of an existing condition.

### Administration

HYBRID 2 (SEQ ID NO:3), as described herein can be administered separately to the same subject, optionally sequentially, or can be co-administered simultaneously as a pharmaceutical or immunogenic composition.

The active ingredients can be administered to a patient in need of treatment via any suitable route. The precise dose will depend upon a number of factors, as is discussed below in more detail.

Some suitable routes of administration include (but are not limited to) oral, rectal, nasal, topical (including buccal and sublingual), vaginal or parenteral (including subcutaneous, intramuscular, intravenous, intradermal, intrathecal and epidural) administration, or administration via oral or nasal inhalation.

The composition is deliverable as an injectable composition, is administered orally, is administered to the lungs as an aerosol via oral or nasal inhalation.

One suitable route of administration is sublingually, e.g. applied under the subject's tongue.

For administration via the oral or nasal inhalation routes, preferably the active ingredient will be in a suitable pharmaceutical formulation and may be delivered using a mechanical form including, but not restricted to an inhaler or nebuliser device.

Further, where the oral or nasal inhalation routes are used, administration by a SPAG (small particulate aerosol generator) may be used.

For intravenous injection, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

Pharmaceutical compositions for oral administration may be in tablet, capsule, powder or liquid form. A tablet may comprise a solid carrier such as gelatin or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

The composition may also be administered via microspheres, liposomes, other microparticulate delivery systems or sustained release formulations placed in certain tissues including blood. Suitable examples of sustained release carriers include semipermeable polymer matrices in the form of shared articles, e.g. suppositories or microcapsules. Implantable or microcapsular sustained release matrices include polylactides copolymers of L-glutamic acid and gamma ethyl-L-glutamate, poly (2-hydroxyethyl-methacrylate) or ethylene vinyl acetate.

Examples of the techniques and protocols mentioned above and other techniques and protocols which may be used in accordance with the invention can be found in Remington's Pharmaceutical Sciences, 16th edition, Oslo, A. (ed), 1980.

### Pharmaceutical Compositions

As described above, the present invention extends to a pharmaceutical composition for the treatment of a coronavirus infection.

Pharmaceutical compositions according to the present invention, and for use in accordance with the present invention, may comprise, in addition to an active ingredient, a pharmaceutically acceptable excipient, carrier, buffer stabiliser or other materials well known to those skilled in the art. Such materials should be non-toxic and should not interfere with the efficacy of the active ingredient. The precise nature of the carrier or other material will depend on the route of administration, which may be, for example, oral, intravenous, intranasal or via oral or nasal inhalation. The formulation may be a liquid, for example, a physiologic salt solution containing non-phosphate buffer at pH 6.8-7.6, or a lyophilised or freeze-dried powder.

### Dose

The composition is preferably administered to an individual in a "therapeutically effective amount" or a "desired amount", this being sufficient to show benefit to the individual. As defined herein, the term an "effective amount" means an amount necessary to at least partly obtain the desired response, or to delay the onset or inhibit progression or halt altogether the onset or progression of a particular condition being treated. The amount varies depending upon the health and physical condition of the subject being treated, the taxonomic group of the subject being treated, the degree of protection desired, the formulation of the composition, the assessment of the medical situation and other relevant factors. It is expected that the amount will fall in a relatively broad range, which may be determined through routine trials. Prescription of treatment, e.g. decisions on dosage etc., is ultimately within the responsibility and at the discretion of general practitioners, physicians or other medical doctors, and typically takes account of the disorder to be treated, the condition of the individual patient, the site of delivery, the method of administration and other factors known to practitioners. The optimal dose can be determined by physicians based on a number of parameters including, for example, age, sex, weight, severity of the condition being treated, the active ingredient being administered and the route of administration. A broad range of doses may be applicable. Considering oral administration to a human patient, for example, from about 10 µg to about 1000 µg of agent may be administered per human dose, optionally for 3 to 4 doses. Dosage regimes may be adjusted to provide the optimum therapeutic response and reduce side effects. For example, several divided doses may be administered daily, weekly, monthly or other suitable time intervals or the dose may be proportionally reduced as indicated by the exigencies of the situation.

Unless otherwise defined, all technical and scientific terms used herein have the meaning commonly understood by a person who is skilled in the art in the field of the present invention.

### Brief description of the Figures

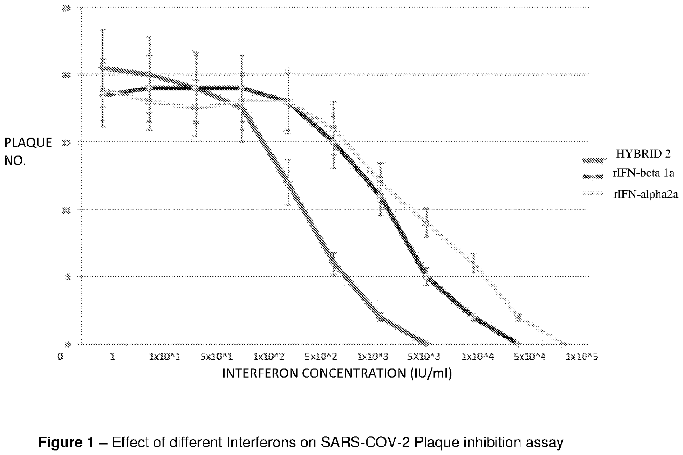

Figure 1 shows results of Plaque Inhibition Assays using Vero Cells of studies comparing three interferons - HYBRID 2, INTERFERON-BETA 1a and_INTERFERON -ALPHA 2a - this gives a comparison based again on Alpha-2a of the other 'INTERFERONS' of 20X, 10X, 5X more potent than Alpha-2a.

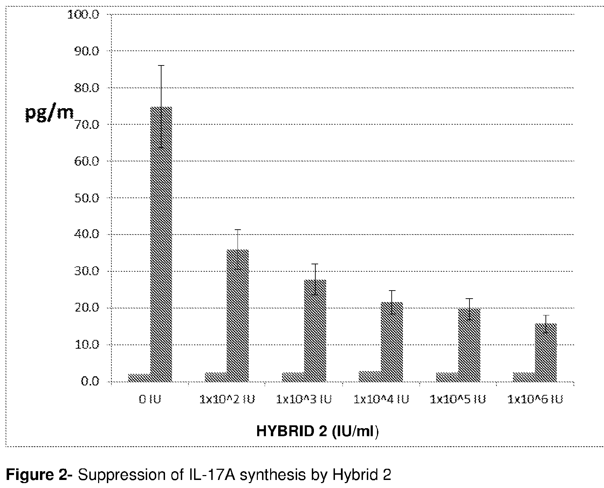

Figure 2 shows suppression of IL-17A synthesis by HYBRID 2.

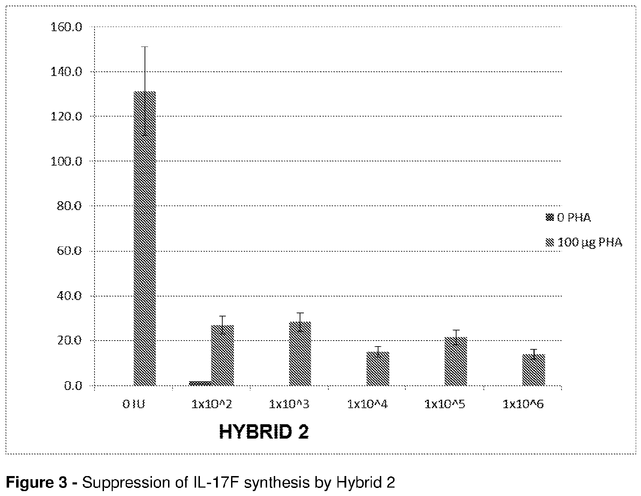

Figure 3 shows suppression of IL 17F with HYBRID 2.

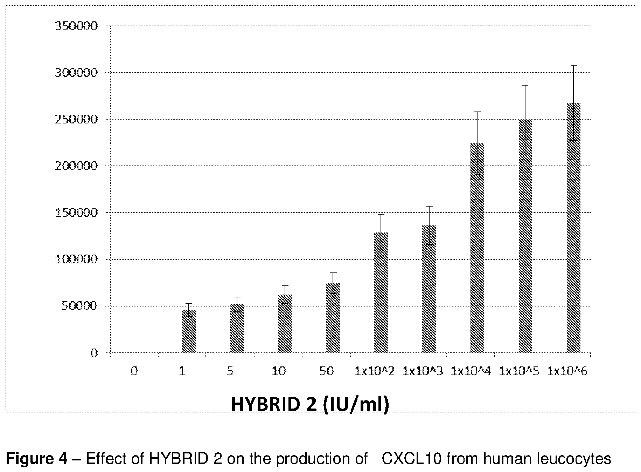

Figure 4 shows the effect of Hybrid 2 on the production of CXCL 10 from human leucocytes.

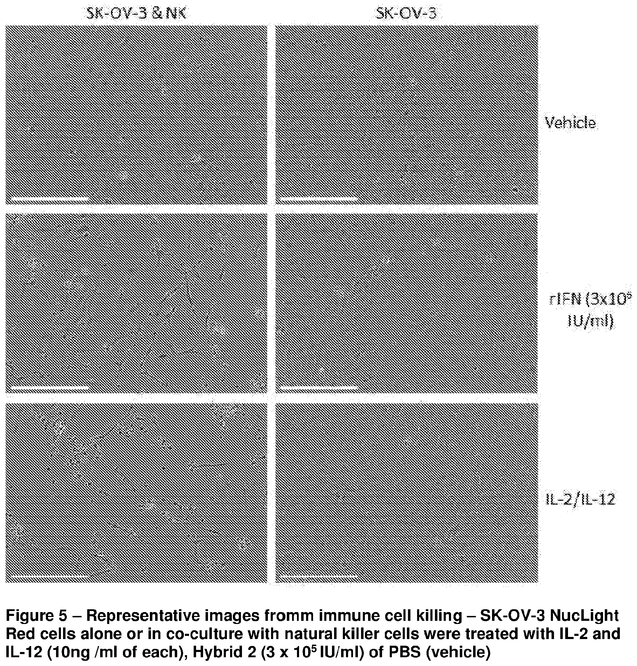

Figure 5 shows images from immune cells and HYBRID 2.
Figure 6 shows HYBRID 2 can induce apoptosis.

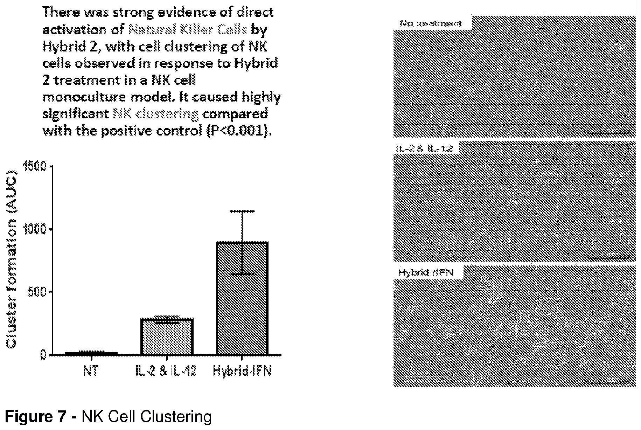

Figure 7 shows NK cell clustering.

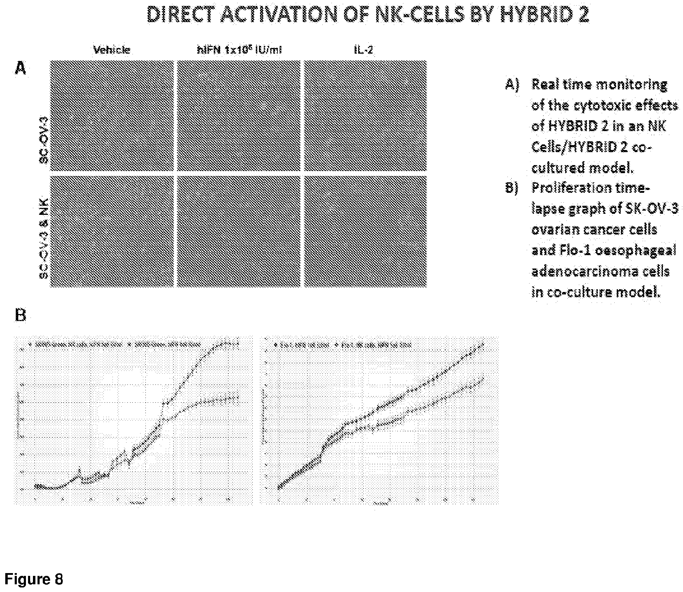

Figure 8 shows direct activation of NK cells by HYBRID 2.

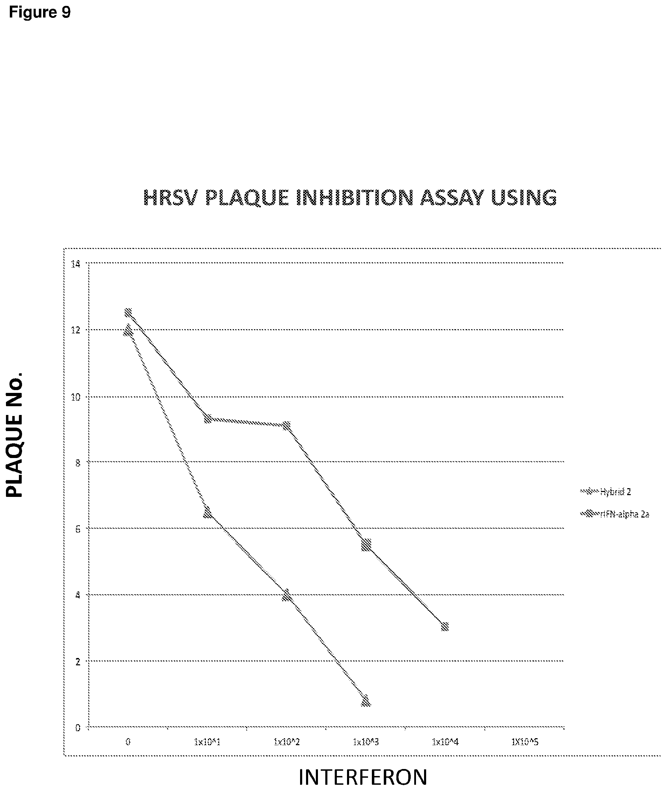

Figure 9 shows HYBRID 2's inhibition of a plaque assay with human RSV.
Figure 10A shows HIV-1 LAI Infectivity by Interferon-alpha 2a (sample A) and HYBRID 2 (sample B) and control inhibitors (RLU).
Figure 10B shows HIV-1 LAI Infectivity Assay (percentage values).
Figure 11A and Figure 11B show determination of IC50 values for HIV-1 infectivity for sample A (Figure 11A) and sample B (Figure 11B).
Figure 12A and Figure 12B show determination of IC50 values for HIV-1 infectivity for control antivirals.
Figure 13 shows Viability Assay with HeLa-CD4-βgal cells (percentage values).
Figure 14A and Figure 14B show determination of CC50 values for sample A (Figure 14A) and sample B (Figure 14B) in HeLa-CD4-βgal cells (percentage values).
Figure 15A and Figure 15B show determination of CC50 values for control antivirals with HeLa-CD4-βgal cells (percentage values).
Figure 16 shows HRSV inhibition by interferon-alpha 2a (sample A) and HYBRID 2 (sample B) (A490).
Figure 17 shows HRSV infectivity assay (percentage values).
Figure 18A and Figure 18B show Determination of IC50 values for sample A (Figure 18A) and sample B (Figure 18B) in HRSV infectivity (percentage values).
Figure 19A and Figure 19B show Determination of CC50 values for sample A (Figure 19A) and sample B (Figure 19B) in with HEp-2 cells (percentage values).
Figure 20 shows HIV-1 LAI infectivity by test-items Interferon-alpha 14 (sample A), HYBRID 2 (sample B) and Interferon-beta 1a (sample C) and control inhibitors (RLU).
Figure 21 shows HIV-1 LAI Infectivity Assay (percentage values) for test-items Interferon-alpha 14 (sample A), HYBRID 2 (sample B) and Interferon-beta 1a (sample C).
Figure 22A (Interferon-alpha 14), Figure 22B (HYBRID 2) and Figure 22C (Interferon-beta 1a) show Determination of IC50 values for HIV-1 infectivity.
Figure 23 shows Viability Assay with HeLa-CD4-βgal cells (percentage values] for test-items Interferon-alpha 14 (sample A), HYBRID 2 (sample B) and Interferon-beta 1a (sample C).
Figure 24 shows Determination of CC50 values for sample A (Figure 24A), sample B (Figure 25B) and sample C (Figure 24C) in HeLa-CD4-βgal cells (percentage values).
Figure 25 shows the Tissue Culture Infectious Dose of Herpes Simplex 1 virus in VERO cells when treated with HYBRID 1, HYBRID 2, Interferon alpha-14, Interferon alpha-2a and Interferon beta-1a.
Figure 26 shows the median TCID converted into plaque-forming units (PFU).

### Experimental Data

### Experiment 1: The effect of IFNα-14 and hybrids on the cytopathic effect following infection from SARS-CoV-1 Coronavirus.

The effectiveness of the hybrids to inhibit the cytopathic effect following SARS-CoV-1 infection was tested in a cytopathic endpoint assay. All endpoint assays were carried also out using the multi-subtype Multiferon and IFN-α 14 as well as the anti-viral Ribavirin for comparison.

### Preparation of anti-viral treatments.

A broad range of concentrations (obtained by ten-fold dilutions) encompassing the inhibitory dosages commonly used for other viral-host combinations was tested. Compounds were dissolved Hank's buffered-saline solution.

For plaque assays, 5-fold drug dilutions were prepared using growth media as specified below. SARS-CoV-1 production and infection African Green Monkey (Vero E6) cells (American Type Culture Collection, Manassas, VA, USA) were propagated in 75cm cell culture flasks containing growth medium consisting of Medium 199 (Sigma, St Louis, USA) supplemented with 10% foetal calf serum (FCS; Biological Industries, Israel). SARS-HCoV2003VA2774 (an isolate from a SARS patient in Singapore) was propagated in Vero E6 cells. Briefly, 2 ml of stock virus was added to a confluent monolayer of Vero E6 cells and incubated at 37°C in 5% C0₂ for one hour. 13 ml Medium 199, supplemented with 5% FCS, was then added. The cultures were incubated at 37°C in 5% C0₂ and the inhibition of the cytopathic effect gauged by observing each well through an inverted microscope. Where 75% or greater inhibition was observed after 48 hours, the supernatant was harvested. The supernatant was clarified at 2500 rpm and then aliquoted into cryovials and stored at -80°C until use.

### Virus handling and titration

Virus titre in the frozen culture supernatant was determined using a plaque assay carried out in duplicate. Briefly, 100 microlitres of virus in a 10-fold serial dilution was added to a monolayer of Vero E6 cells in a 24 well-plate. After incubation for an hour at 37°C in 5% C0₂, the viral Medium 199 supplemented with 5% FCS was added. Cells were fixed with 10%(v/v) formalin and stained with 2% (w/v) crystal violet. The plaques were counted visually and the virus titre in plaque forming units per ml (pfu/ml) calculated.

### Cytopathic endpoint assay

The effect of each anti-viral treatment was tested in quadruplicate. Briefly, 100 microlitres of serial 10-fold dilutions of each treatment were incubated with 100 microlitres of Vero E6 cells giving a final cell count of 20,000 cells per well in a 96-well plate. Incubation was at 37°C in 5% C0₂ overnight for the interferon preparations and for one hour for the of infection (MOI) (virus particles per cell) of 0. 5. The plates were incubated at 37°C in 5% C0₂ for three days and the plates were observed daily for cytopathic effects. The end point was the diluted concentration that inhibited the cytopathic effect in all four set-ups.

To determine cytotoxicity, 100 microlitres of serial 10-fold dilutions of each of the treatments were incubated with 100 microlitres of Vero E6 cells giving a final cell count of 20,000 cells per well in a 96-well plate, withoutviral challenge. The plates were then incubated at 37°C in 5% C0₂ for three days and toxicity effects were observed for using an inverted microscope.

10 microlitres of virus at a concentration of 10,000 pfu/well were then added to each test well. This equates to a multiplicity of infection (MOI) (virus particles per cell) of 0.5. The plates were incubated at 37°C in 5% C0₂ for three days and the plates were observed daily for cytopathic effects. The end point was the diluted concentration that inhibited the cytopathic effect in all four set-ups (CIA100).

To determine cytotoxicity, 100 microlitres of serial 10-fold dilutions of each treatment were incubated with 100 microlitres of Vero E6 cells giving a final cell count of 20,000 cells per well in a 96-well plate, without viral challenge. The plates were then incubated at 37°C in 5% C0₂ for three days and toxicity effects were observed for using an inverted microscope. Interferons which showed complete inhibition were tested further at the lower viral titres of 10³ and10² pfu/well.

### Experiment 2: The effect of hybrids on plaque formation following infection from SARS-CoV-1 Coronavirus,

The effectiveness of the hybrids to inhibit plaque formation following SARS-CoV-1 infection was tested in a plaque reduction assay.

The plaque assay was performed using 10-fold dilutions of a virus stock, and 0.1 ml aliquots were inoculated onto susceptible cell monolayers. After an incubation period, which allowed virus to attach to cells, the monolayers were covered with a nutrient medium containing a substance, usually agar, that causes the formation of a gel. After plate incubation, the original infected cells released viral progeny. The spread of the new viruses is restricted to neighbouring cells by the gel. Consequently, each infectious particle produced a circular zone of infected cells called a plaque. Eventually the plaque became large enough to be visible to the naked eye. Dyes to stain living cells were used to enhance the contrast between the living cells and the plaques. Only viruses that caused visible damage to cells were assayed in this way i.e. SARS-CoV-1.

These results clearly demonstrate the ability of HYBRID 2 to inhibit the cytopathic effect and plaque formation caused as a result of infection with SARS-CoV-1. The results demonstrate the inhibition of the cytopathic effect in comparison with the anti-viral compound Ribavirin and the multi-subtype interferon Multiferon^{™}.

### Experiment 3: Assays in relation IL-17, Granzyme B, Interferon gamma, Interferon alpha, NK cells

Assays utilising interferon alpha 14 and Hybrid 2 in relation to key immune modulators were undertaken as would be understood in the art.

### Effect on HYBRID 2 on immune cell killing

The effect of a hybrid recombinant interferon was tested in a live cell imaging assay of immune cell killing on an IncuCyte ZOOM platform.

SK-OV-3 ovarian cancer cells with red labelled nuclei (SK-OV-3 NucLight Red) were used as target cells in the study. For immune cell killing, the cells were co-cultured with natural killer (NK) cells and positive controls consisted of cells treated with IL-2 and IL-12. Apoptosis was detected by staining caspase 3/7 positive objects while cell number was determined by counting of red nuclei. The results from the co-culture model were compared to those of a mono-culture model consisting of SK-OV-3 NucLight Red cells alone.

An initial optimisation experiment was carried out that tested 4 ratios of target and effector cells. These results determined that 5,000 natural killer cells and 2,000 SK- OV-3 NucLight Red cells per well of a 96-well plate gave a suitable assay window for detecting immune cell killing.

Eight doses of hybrid recombinant interferon ranging from 10 IU/ml to 3×10⁶ IU/ml were tested for effects on immune cell killing. Results were compared to no treatment controls, vehicle treated controls and IL-2/IL-12 treated cells. All conditions were tested using cells in co-culture (SK-OV-3 NucLight and NK cells) and mono-culture (SK-OV-3 NucLight Red).

Cells were monitored for 4 days using an IncuCyte ZOOM, and IncuCyte software was used to measure green (apoptosis) and red (cell number) object count over time. Area under the curve (AUC) analysis was used to quantitate apoptosis and cell number over the time course.

IL-17, Il-6, CCL-5 and the immune response have been determined to be modulated following Covid-19 infection, in particular Covid-19 induced Acute Respiratory Distress Syndrome where IL-17 inflammation has been indicated as being significant.

Hybrid recombinant interferon caused a strong induction in apoptosis in the co-culture model, with an increase in AUC from 200 in vehicle controls to 1174 at the top dose of hybrid recombinant interferon. An EC₅₀ of 1.5×10⁶ IU/ml was derived for apoptosis induction. In contrast, cells in mono-culture displayed only a marginal response to hybrid rIFN.

Hybrid recombinant interferon also caused a reduction in cell number. AUC values for cell number fell from 39921 in vehicle controls to 19501 at the top dose of hybrid rIFN. An IC₅₀ value of 1.3×10⁶ IU/ml was determined for the reduction in cell number.

There was evidence of very strong direct activation of natural killer cells by hybrid rIFN, with cell clustering of NK cells observed in response to hybrid recombinant interferon treatment in a NK cell monoculture model.

Hybrid 2 has been demonstrated to modulate Il-17 (inhibits both 17 A and F very strongly), Il-6, CCL-5, and CCl-2 and to provide an antiviral NK response.

### Experiment 4: Evaluation of antiviral activity of Interferon-alpha 2a (sample A) and HYBRID 2 (sample B) against HIV and HRSV

Full-dose antiviral testing was carried out on test items Interferon-alpha 2a (sample A) and HYBRID 2 (sample B). These items were tested with human immunodeficiency virus type 1 (HIV-1) LAI strain and human respiratory syncytial virus (HRSV) Long strain. All test-items were provided in solid from which 0.25mg/mL (sample A) and 0.10mg/mL (sample B) were prepared in ddH2O, aliquoted, and stored at -80C to prevent repeated freeze thaw cycles.

The project utilized different adherent cell lines to evaluate the antiviral activity of the test-items against different viruses. Standard assays run at RVX were used for each virus. In brief, test-items were either pre-incubated with the target cells (HIV assay), and for the HRSV assay, the putative inhibitors were pre-incubated with virus for 30 min before adding the virus and inhibitor mix to the cells. Inhibitors were present in the cell culture medium for the duration of the infection (see below). For each antiviral assay, a viability test was set up in parallel using the same concentrations of inhibitors tested in the antiviral assays. Viability assays were used to determine compound-induced cytotoxicity effects in the absence of virus. Cell viability was determined by the XTT method. Viability assays were conducted for the same periods of time evaluated in the corresponding antiviral assays. Nine concentrations for HIV-1 and eight concentrations for HRSV of the test-items were evaluated in duplicates. Ten-fold serial dilutions starting at 10µg/mL were evaluated. When possible, IC50 and CC50 values for the inhibitors were determined for each assay using *GraphPad Prism* software.

### HIV-1 assay:

Antiviral activity against HIV was evaluated using relative luminescence generated with a kit to reveal beta-galactosidase activity. To determine activity against HIV we used HeLa-CD4-betagal cells in which infection with HIV induces expression of beta-galactosidase. The extent of infection was monitored after 2 days of infection.

### HRSV assay:

Antiviral activity against HRSV using Hep-2 cells was evaluated with an immunoassay to monitor expression of viral antigens in cells infected with the virus. Cells were challenged with virus in the presence of different concentrations of control or test inhibitors. The extent of infection was monitored after 3 days of infection by quantifying the levels of viral antigens with a colorimetric readout.

### Antiviral activities of test-items A (Interferon-alpha 2a) and B (HYBRID 2)

Overall, test-items *A* and *B* displayed antiviral activity against HIV-LAI and HRSV-Long. IC50 values against HIV were 0.3ng/mL and 0.009ng/mL, for A and B, respectively, whereas IC50 values against HRSV were 0.2ng/mL and 0.03ng/mL, respectively (*Table 1*).

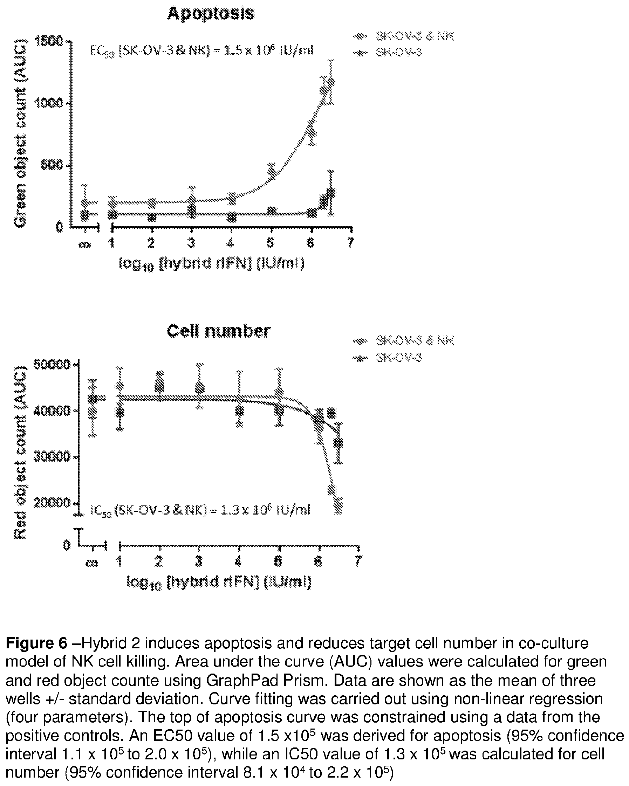

A loss of cell viability was observed in HeLa-CD4-beta-gal cells treated with both test-items. The highest inhibition of cell viability (observed at 10µg/mL) did not affect more than 30-33% of the cell culture (*Figure 6**, page 15*), and the selectivity indices for anti-HIV activity were extremely high in both cases. Of note, it is important to note that none of the inhibitors blocked completely HIV infection. It is possible that a fraction of the culture (estimated at about 25%) was not sensitive to the activity of the inhibitors. However, control inhibitors evaluated in parallel completely block HIV infection. A similar profile (lack of complete inhibition) was also observed in the anti-HRSV assays, but this time the inhibitors block approximately 94%. It is also important to note that because of the lack of complete inhibition, the IC50 values generated by GraphPad Prism may differ from the concentrations at which 50% of the inhibitory effect is observed.

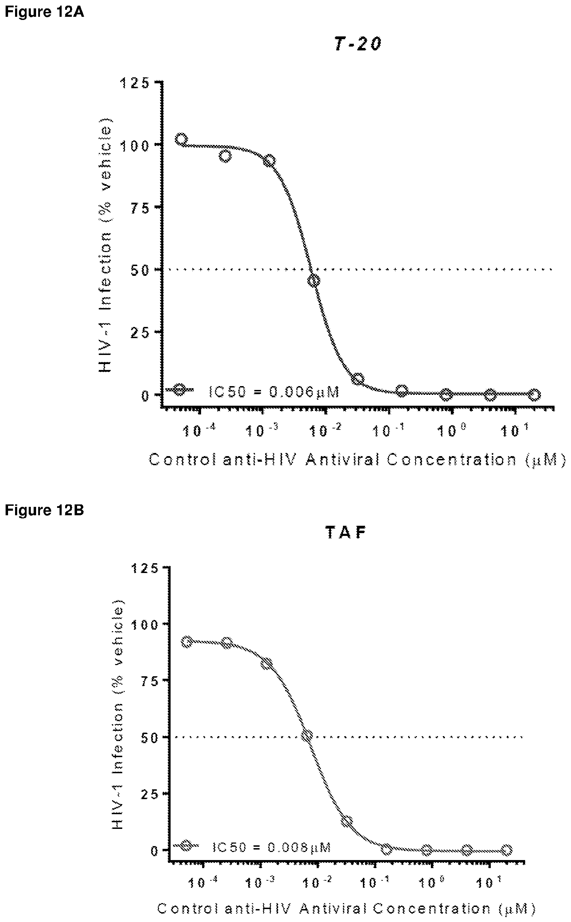

Compound-induced cytotoxicity was also observed with HEp-2 cells, but the loss of cell viability never reached 50 percent of the culture even at the highest concentration tested (10µg/mL) (*Figure 12**, page 23*). Excellent selectivity indices were also observed with HRSV.

As shown below, multiple antiviral inhibitors were used as controls and run in parallel with test-items *A and B.* Inhibitor controls included in the studies were *TAF* (*tenofovir alafenamide*) a nucleotide reverse transcriptase inhibitor of HIV, *T20* (*enfuvirtide*) a fusion inhibitor peptide that blocks HIV entry, and *ribavirin,* a guanosine analog with broad antiviral activity, including against HRSV. All the inhibitors blocked the respective target viruses as expected, and validated the sensitivity of the assays used in this study.

### Control Inhibitors and Quality Controls

Quality controls for the infectivity assays were performed on every plate to determine: i) signal to background (S/B) values; ii) inhibition by known inhibitors, and iii) variation of the assay, as measured by the coefficient of variation (C.V.) of all replicate test-item data points. All controls worked as anticipated for each assay. Known antivirals for HIV (TAF and T20) blocked infection over 99% at some concentrations tested, and when assessed in full dose response curves they blocked viral replication as reported in literature. The antiviral control used in the HRSV assay, ribavirin, a broad-spectrum antiviral agent blocked infection over 99% at 50µM. The viability control (emetine) used in XTT cytotoxicity assays displayed inhibition greater than 85% for all cell types tested.

Overall variation in the infection assays was 4.4% (HIV) and 4.9% (HRSV), and overall variation in the viability assays was 3.7% (HIV) and 4.6% (HRSV). The signal-to-background (S/B) in the infection assays was 366-fold and 3.4-fold, respectively, in the HIV and HRSV assays. Signal-to-background (S/B) for the viability assays was 10-fold for both assays.

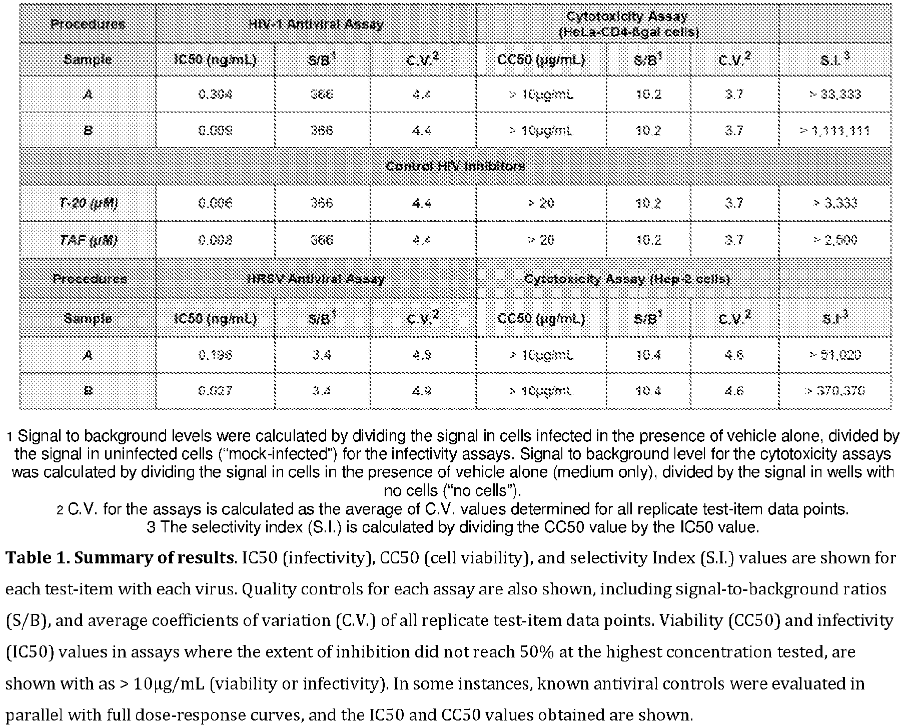

### Experimental Procedures - Antiviral assay with β-galactosidase readout

### HIV-1 Infectivity assay

Infectivity assays were performed by challenging HeLa-CD4-βgal cells with HIV-1 LAI in the presence or absence of test-items. HeLa-CD4-βgal cells express beta-galactosidase under the control of the HIV-1 LTR promoter. Upon infection with HIV and expression of the viral Tat protein beta-galactosidase expression is induced. HeLa-CD4-βgal cells were seeded at 12,000 cells per well in a white TC-treated 96-well flat bottom plate and maintained in DMEM supplemented with 10% fetal bovine serum (FBS), hereby called DMEM10. Test-items were diluted 10-fold in U-bottom plates using DMEM10. Test material dilutions were prepared at 1.25X the final concentration. Cells were incubated with the 1.25X-diluted testmaterial (80µL per well) for 30 minutes at 37°C in 5% CO2. Following the test material pre-incubation, HIV-LAI virus prepared in DMEM10 was added to the cells (20µL per well) and plates were incubated at 37°C in a humidified incubator with 5% CO2 for 48 hours. Each well contained 100µL final volume. The volume of virus used in the assay was previously determined to produce a signal in the linear range inhibited by TAF and T20, known HIV-1 inhibitors. Infections were performed in duplicate reactions. After two days of infection, cells were monitored for beta-galactosidase activity using the Galacton-Star^{®} kit (*ThermoFisher*). The Galacto-Star test is a sensitive chemiluminescent assay that detects beta-galactosidase in mammalian cell lysates. Luminescence was measured with a 1-second readout. Nine data points from duplicate of serial ten-fold dilutions of the test-items were evaluated ranging from 0.0001 ng/mL to 10µg/mL, were evaluated. Controls included cells incubated with no virus ("mock-infected"), infected and incubated with vehicle alone (DMEM10), or infected in the presence of *TAF* and *T20* (known inhibitors of HIV infection) in a full dose response starting from 20µM (single data points) or at 0.5µM.

### QC HIV-1 infectivity assay

The average S/B in the assay was 366 (background determined with "mock-infected" cells), whereas the average variation for all data points was 4.4% (C.V. values) and 3.6% (C.V. values for all wells displaying greater than 50% infection). Background levels observed in mock-infected cells in the absence of virus were subtracted from all samples. Control antivirals used in this assay (TAF 0.5µM and T-20 0.5µM) blocked infection near 100%.

### Experimental Procedures - Antiviral assay (Immunostaining procedure)

To determine antiviral activity against human respiratory syncytial virus (HRSV) an immunostaining assay was used to monitor the extent of infection. In this type of assay, infected cells are fixed and then a cocktail of anti-RSV antibodies is used to quantify the amount of viral antigen using a colorimetric readout.

### HRSV infectivity assay

For the HRSV infectivity assay we used the Long strain of HRSV to infect HEp-2 cells (human cervix epithelial adenocarcinoma). Cells were maintained in MEM with 10% fetal bovine serum (FBS) for the seeding procedure. The day before infection, cells were seeded at 12,000 cells per well in a 96-well clear flat bottom plate and incubated at 37°C for 24 hours. The day of infection, test-items were serially diluted (10-fold) in a U-bottom plate using MEM with 2% fetal bovine serum (FBS), hereby called MEM2. Dilutions were prepared at 2X the final concentration. Equal volume (30µL) of Long virus diluted in MEM2 was incubated with 30µL of 2X concentrated test-items for 30 minutes at room temperature. The volume of virus used in the assay was previously determined to produce a signal in the linear range inhibited by *ribavirin,* a prodrug that is metabolized into nucleoside analogs that blocks viral RNA synthesis and viral mRNA capping. Following the 30-minute pre-incubation, cells were washed with MEM2, then 50µL of the virus/sample mixture was added to the cells and the plate was incubated at 37°C in a humidified incubator with 5% CO2 for 1 hour. After allowing viral entry, an additional volume of the corresponding test-items or control inhibitor in MEM2 was added to each well. Incubation was carried out for 3 days at 37°C in a humidified incubator with 5% CO2.

Test-items were evaluated in duplicates using serial 10-fold dilutions in MEM2. Controls included cells incubated with no virus ("mock-infected"), infected and incubated with vehicle alone (MEM2), and infected in the presence of *ribavirin* (broad-spectrum antiviral) at 50µM. After 3 days of infection, cells were stained with an immunostaining protocol using a cocktail of different anti-HRSV antibodies to quantify infection levels. Cells were then washed and fixed and the amount of viral antigen was estimated with an HRSV-specific immunostaining assay utilizing a cocktail of mouse monoclonal antibodies directed against several viral antigens. A set of controls were run on each 96-well plate, including cells incubated with virus in the presence of vehicle alone, in the presence of a control inhibitor, ribavirin, or cells incubated in the absence of virus ("mock-infected" control) to determine background levels of the assay. Background was subtracted from all data points before calculating the percent activity as compared to the vehicle alone.

### QC HRSV infectivity assay

Infectivity was determined by monitoring the absorbance at 490 nm. The signal-to-background ratio (S/B) in the assay was 3.4, determined as the percentage of infected cells treated with MEM2 only compared to that of "mock-infected" cells. The average variation for all replicate test-item data points was 4.9% (average of all C.V. values), and 6.3% (C.V. values for all test-item wells displaying greater than 50% infection).

### Experimental Procedure - Cytotoxicity assays

### Viability assay (XTT) to assess compound-induced cytotoxicity

Uninfected cells were incubated with test-items or control viability inhibitor dilutions using one dose higher test-item concentrations as those used in the infectivity assay. The incubation temperature and duration of the incubation period mirrored the conditions of the corresponding infectivity assay. Cell viability was evaluated with the XTT method. The tetrazolium salt (XTT) is cleaved to an orange formazan dye throughout a reaction that occurs only in viable cells with active mitochondria. The formazan dye is directly quantified using a scanning multi-well spectrophotometer. Background levels obtained from wells with no cells were subtracted from all data-points. The extent of viability was monitored by measuring absorbance at 490 nm.

### QC and analysis of cytotoxicity data

The average signal obtained in wells with no cells was subtracted from all samples. Readout values were given as a percentage of the average signal observed in uninfected cells treated with vehicle alone (medium only). The signal-to-background (S/B) obtained were 10.2 (HeLa-CD4-βgal cells incubated 48 hours), 10.4 (HEp-2 cells incubated 3 days). *Emetine* was used as a cytotoxic compound control in the viability assays and inhibited cell viability greater than 90% at 1µM.

### Results - Human immunodeficiency virus type 1 (HIV-1) antiviral activity

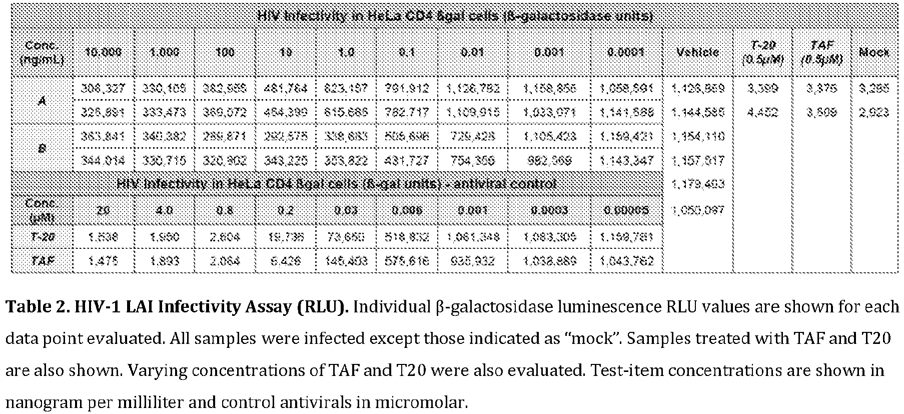

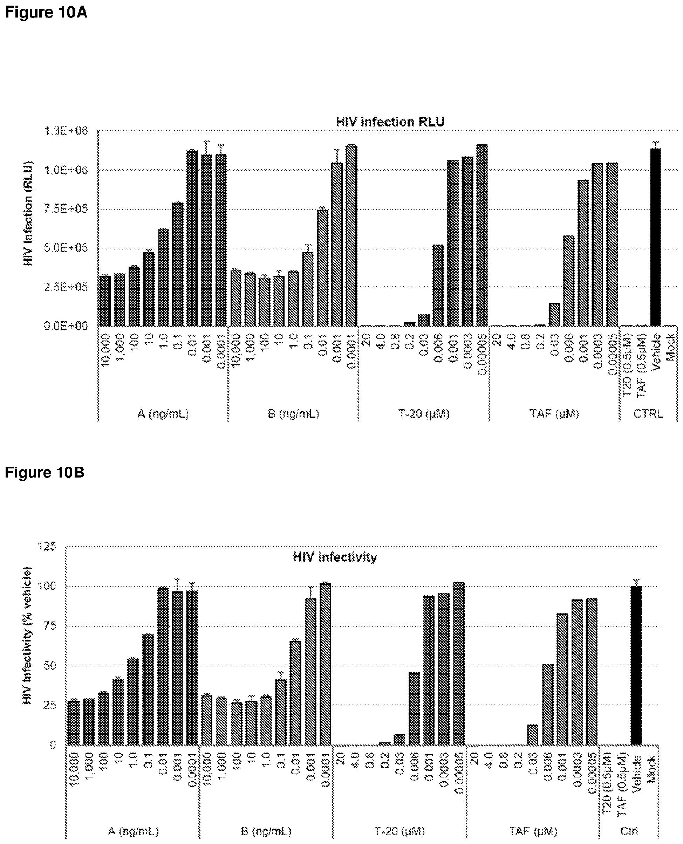

**Figure 10A** **shows HIV-1 LAI Infectivity by sample A (Interferon-alpha 2a) and sample B (HYBRID 2) and control inhibitors (RLU).** Data is shown as relative luminescence units (RLU) values in wells containing HeLa-CD4-βgal cells infected in the presence of either vehicle alone or varying concentrations of test-items (average of duplicates with standard deviation). Uninfected cells are shown as "Mock". *TAF* and *T20* are included as antiviral controls (single data points in full dose response or average of duplicate data points at 0.5µM).

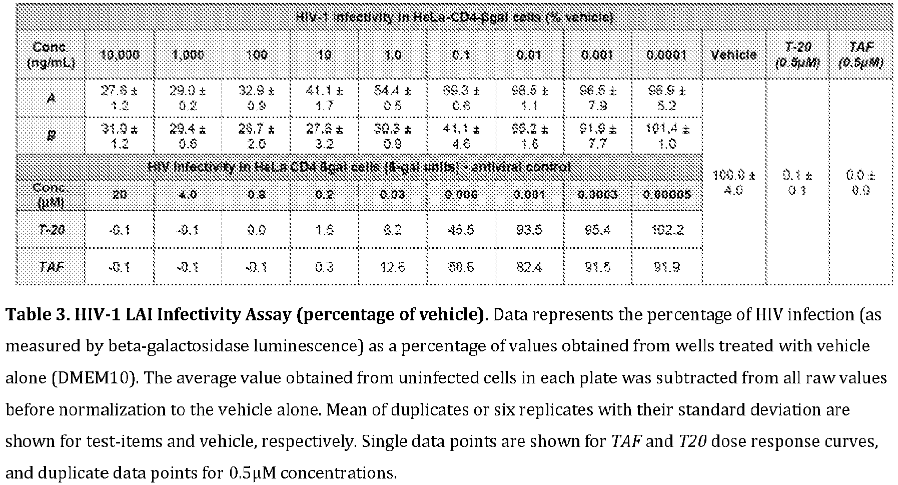

**Figure 10B** **shows HIV-1 LAI Infectivity Assay (percentage values).** Results show the extent of HIV-1 LAI infection, as determined by a beta-galactosidase assay (luminescence readout) at 48 hours. Data is normalized to the activity observed in cells in the absence of test-item (vehicle alone). Test-item results show the average of duplicate data points with the standard deviation (s.d.). Also included, the dose-response observed with *TAF* and *T20* (single data-points).

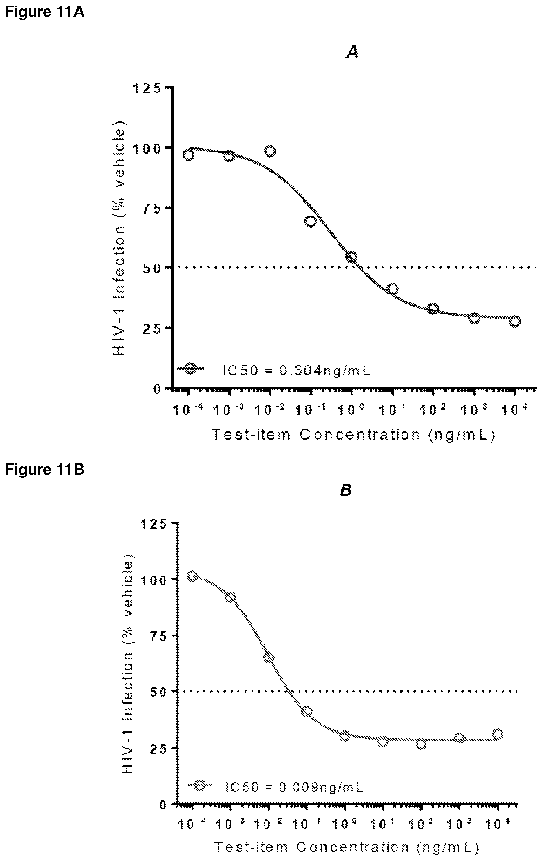

**Figure 11A and Figure 11B** **show Determination of IC50 values for HIV-1 infectivity for sample A (****Figure 11A****) and sample B (****Figure 11B****).** Values indicate the percentage of HIV infectivity compared to samples incubated with vehicle alone. Results show the average of duplicate data points with the standard deviation (s.d.). Data was adjusted to a sigmoid function and IC50 values were calculated using *GraphPad Prism* software fitting a dose-response curve with a variable slope (four parameters). IC50 values are summarized in *Table 1.*

**Figure 12A and Figure 12B** **show Determination of IC50 values for HIV-1 infectivity for control antivirals.** Values indicate the percentage of HIV-1 infectivity compared to samples incubated with vehicle alone. Results show single data points for control antivirals. Data was adjusted to a sigmoid function and IC50 values were calculated using *GraphPad Prism* software fitting a dose-response curve with a variable slope (four parameters). IC50 values are summarized in *Table 1.*

### Results - Cytotoxicity for human immunodeficiency virus type 1 (HIV-1) assay

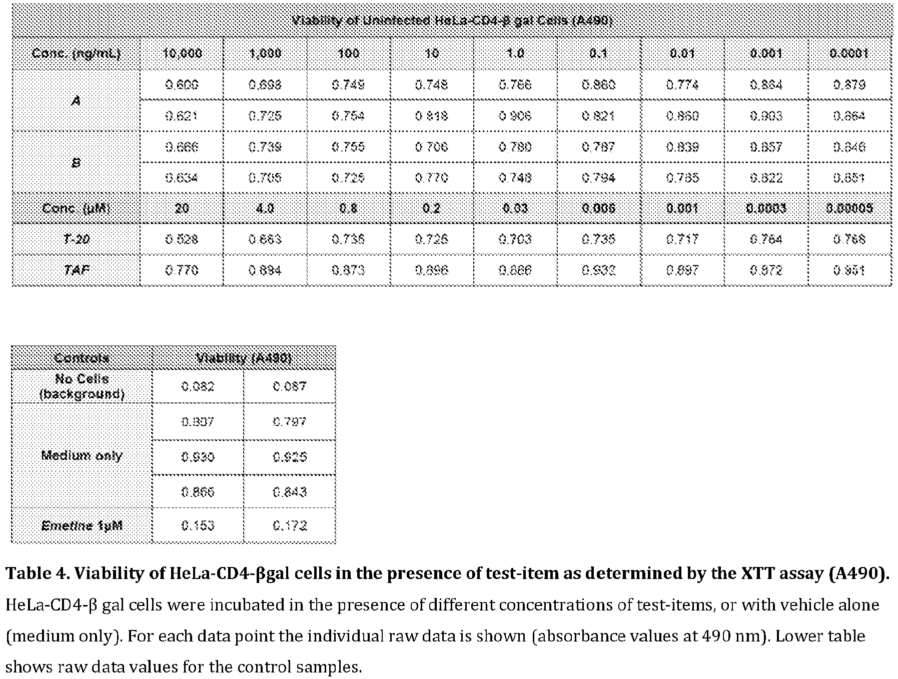

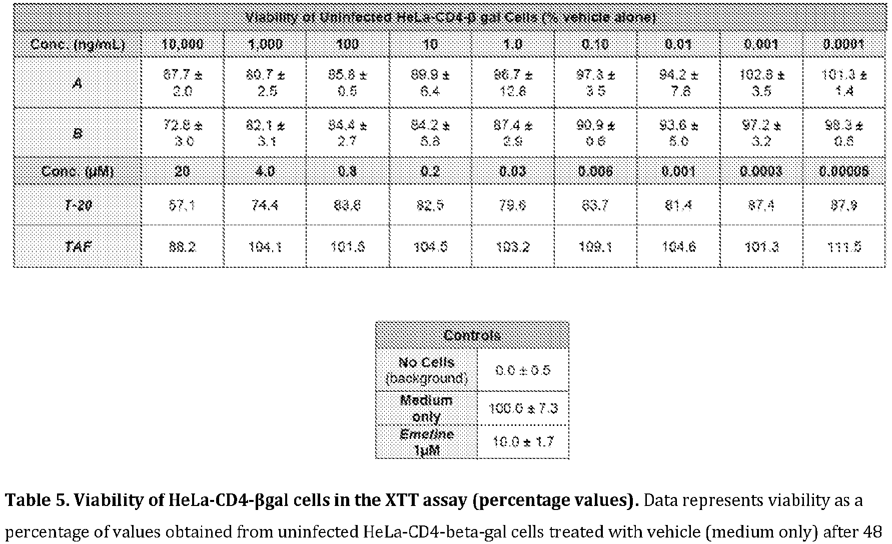

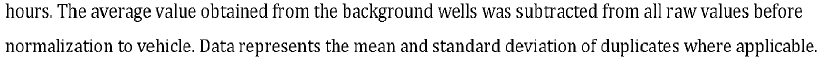

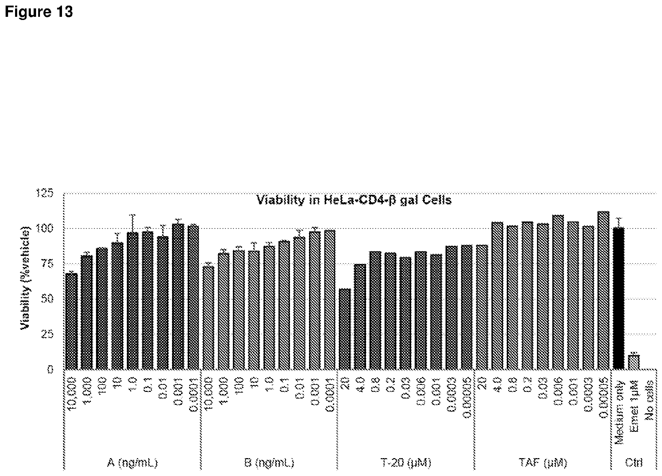

**Figure 13** **shows Viability Assay with HeLa-CD4-βgal cells (percentage values). Results** show the extent of compound-induced cytotoxicity in HeLa-CD4-beta-gal cells incubated for 48 hours, as determined by an XTT readout for viability (absorbance 490 nm readout). Data is normalized to the values observed in cells in the absence of test-item vehicle alone (medium only). Results show the average of duplicate data points with the standard deviation (s.d) for test-items. Also included, the dose-response observed with *TAF* and *T20* (single data-points).

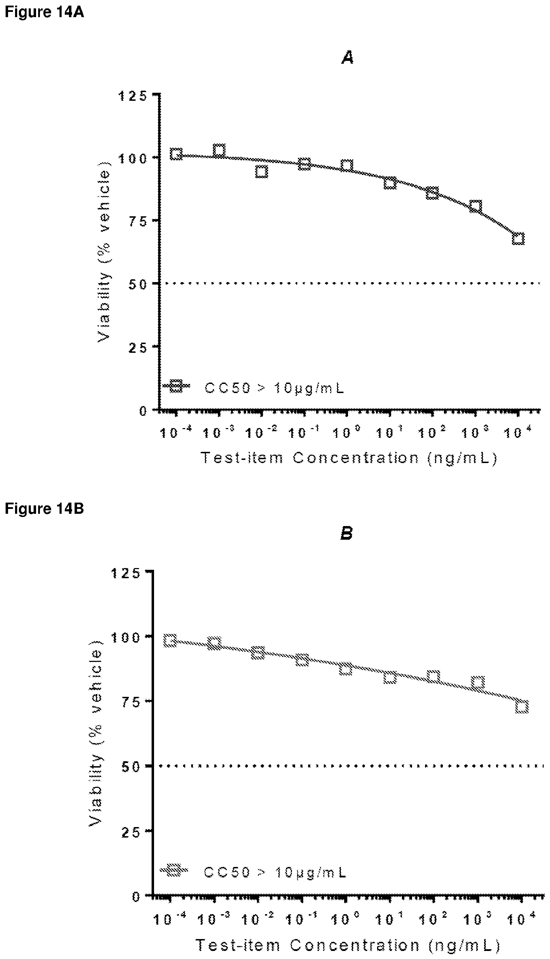

**Figure 14A and Figure 14B** **show Determination of CC50 values for sample A (****Figure 14A****) and sample B (****Figure 14B****) in HeLa-CD4-βgal cells (percentage values).** Results show the extent of test-item-induced cytotoxicity in HeLa-CD4-beta-gal cells incubated for 48 hours, as determined by an XTT readout for viability (absorbance 490 nm readout). Values indicate the percent viability estimated as percentage of that observed in samples incubated with vehicle alone (medium only). Results show the average of duplicate data points. Data was adjusted to a sigmoid function and CC50 values were calculated using *GraphPad Prism* software fitting a dose-response curve with a variable slope (four parameters). When viability did not reach 50%, the CC50 value reported was greater than 10µg/mL. CC50 values are indicated in *Table 1.*

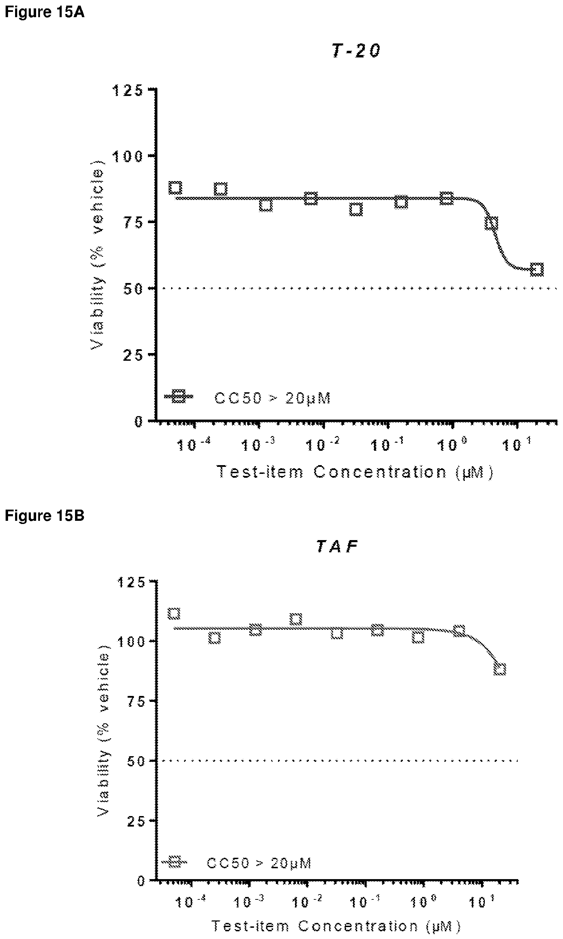

**Figure 15A and Figure 15** **B show Determination of CC50 values for control antivirals with HeLa-CD4-βgal cells (percentage values).** Results show the extent of test-item-induced cytotoxicity in HeLa-CD4-beta-gal cells incubated for 48 hours, as determined by an XTT readout for viability (absorbance 490 nm readout). Values indicate the percent viability estimated as percentage of that observed in samples incubated with vehicle alone (medium only). Results show single data-points. Data was adjusted to a sigmoid function and CC50 values were calculated using *GraphPad Prism* software fitting a dose-response curve with a variable slope (four parameters). When viability did not reach 50%, the CC50 value reported was greater than 20µM. CC50 values are indicated in *Table 1.*

### Results - Human respiratory syncytial virus (HRSV) antiviral activity

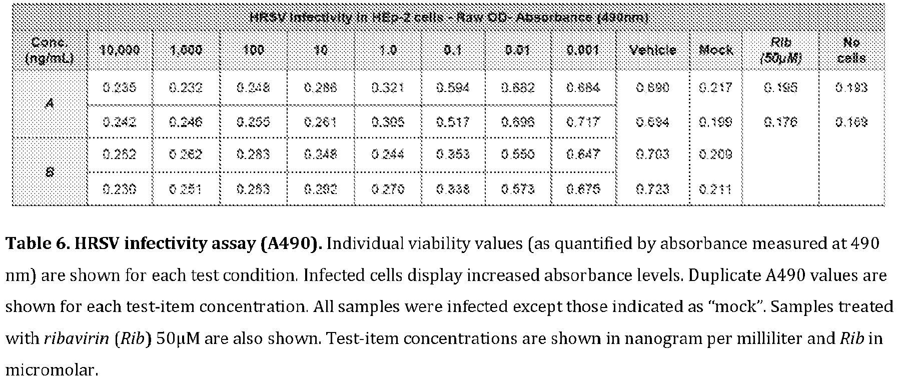

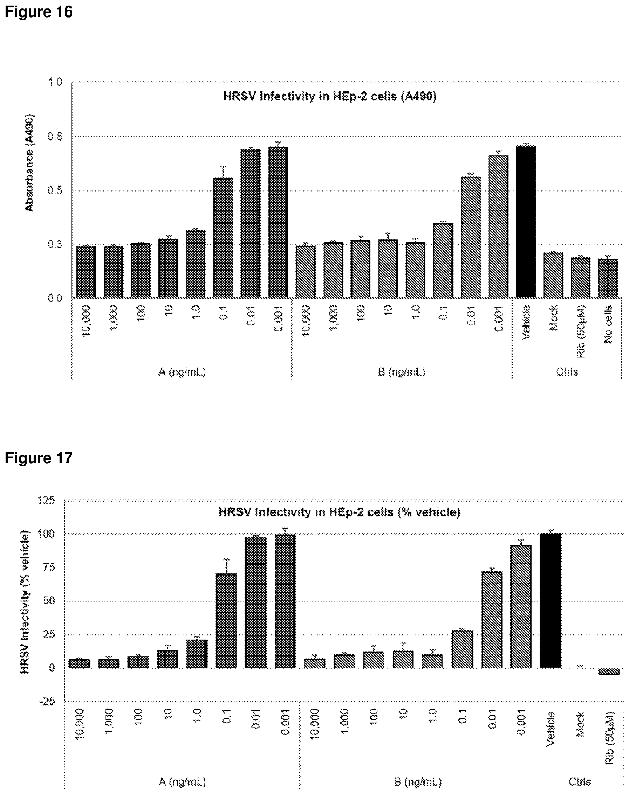

**Figure 16** **shows HRSV inhibition by test-items** (A490). Data is shown as A490 values in wells containing HEp-2 cells infected in the presence of either vehicle alone or varying concentrations of test-items (average of duplicates with standard deviation). Uninfected cells are shown as "Mock". Background levels are shown in wells without cells ("no cells"). *Rib* 50µM is shown as the control antiviral.

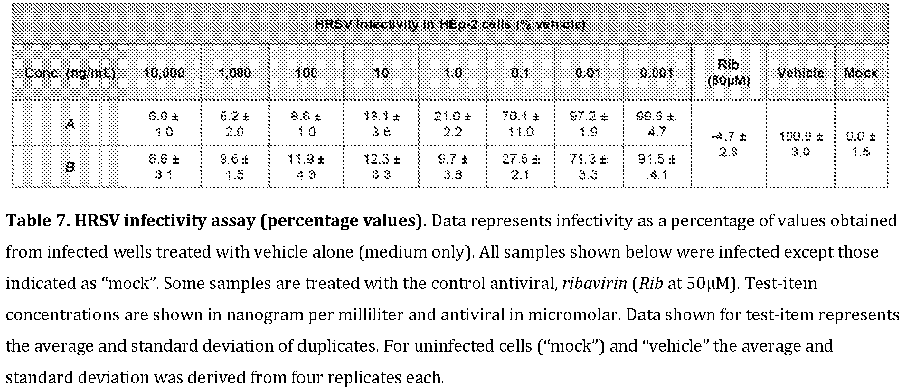

**Figure 17** **shows HRSV infectivity assay (percentage values)**. Results show the extent of HRSV infection, as by an immunostaining readout at 490nm for infectivity after 72 hours. Data is normalized to the activity observed in cells in the absence of test-item (vehicle alone). Test-item results show the average of duplicate data points with the standard deviation.

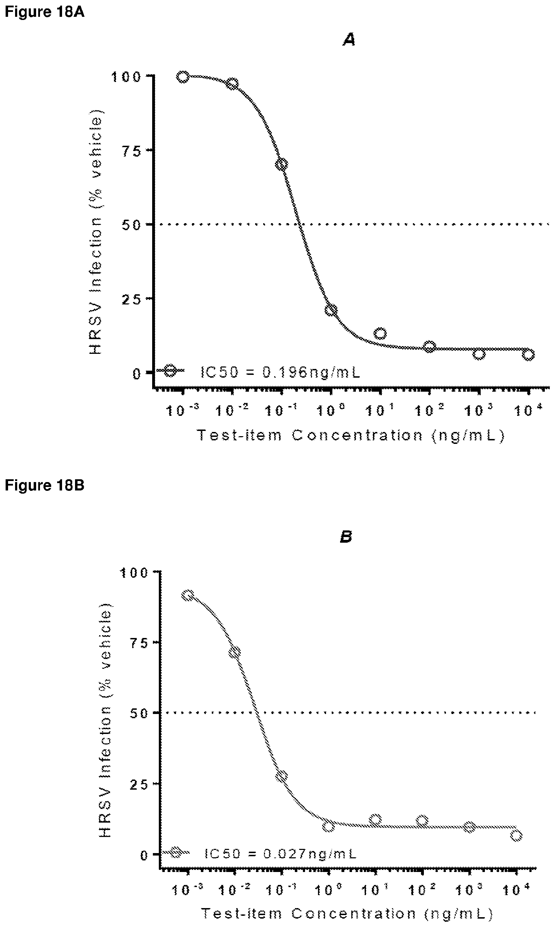

**Figure 18A and Figure 18B** **show Determination of IC50 values for sample A (****Figure 18A****) and sample B (****Figure 18B****) in HRSV infectivity (percentage values).** Results indicate the extent of HRSV infection at 72 hours as compared to samples incubated with vehicle alone (medium only). Results show the average of duplicate data-points. When possible, data was adjusted to a sigmoid function and IC50 values were calculated using *GraphPad Prism* software fitting a dose-response curve with a variable slope (four parameters). IC50 values are summarized in *Table 1.*

### Results - Cytotoxicity for human respiratory syncytial virus (HRSV) assay

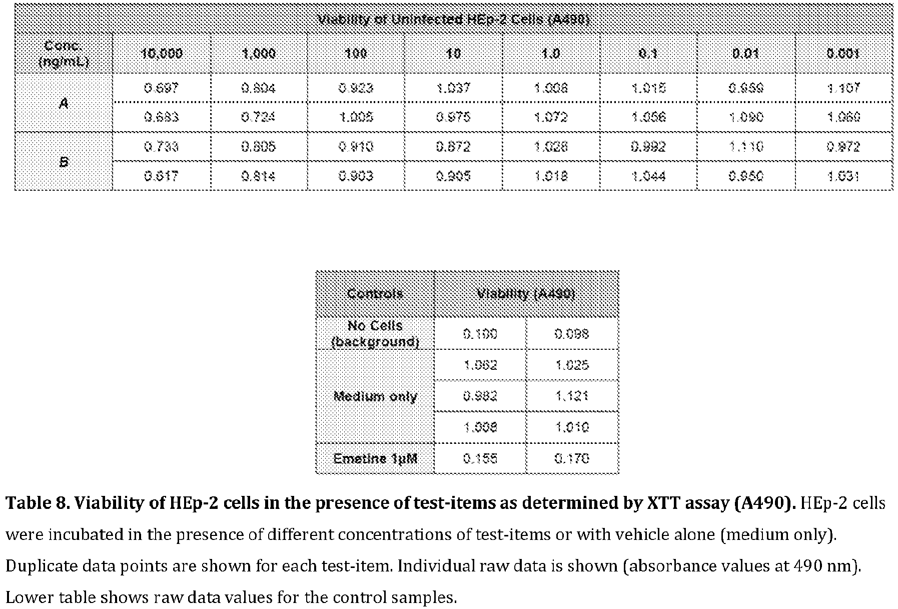

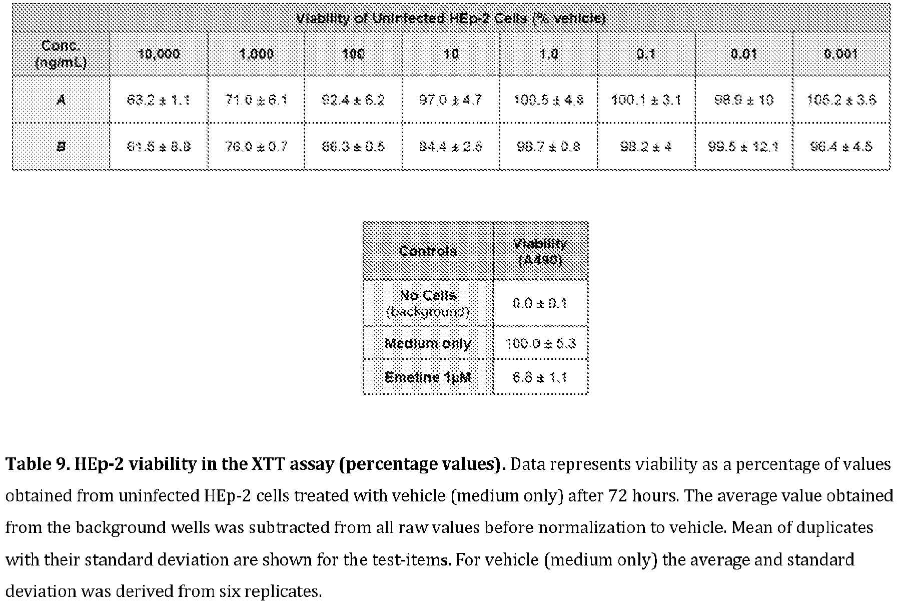

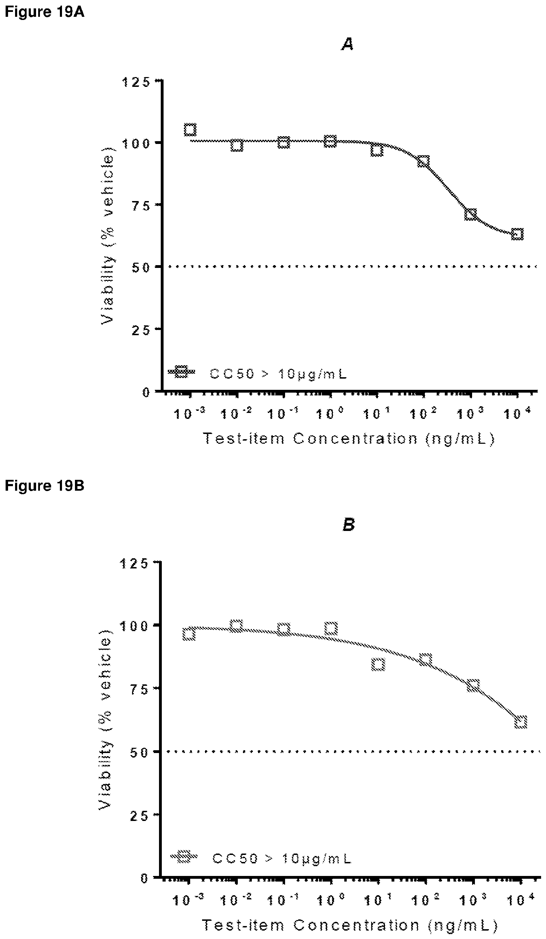

**Figure 19A and Figure 19B** **show Determination of CC50 values for sample A (****Figure 19A****) and sample B (****Figure 19B****) in with HEp-2 cells (percentage values).** Results show the extent of test-item-induced cytotoxicity in HEp-2 cells incubated for 72 hours, as determined by an XTT readout for viability (absorbance 490 nm readout). Values indicate the percent viability estimated as percentage of that observed in samples incubated with vehicle alone (medium only). Data represents duplicate data points for each test-item. Data was adjusted to a sigmoid function and CC50 values were calculated using *GraphPad Prism* software fitting a dose-response curve with a variable slope (4 parameters). When loss of cell viability did not reach 50%, the CC50 value reported was indicated as greater than 10µg/mL. CC50 values also shown in *Table 1.*

### Experiment 5: Evaluation of antiviral activity of Interferon-alpha 14 (sample A), HYBRID 2 (sample B) and Interferon-beta 1a (sample C) against HIV

Full-dose antiviral testing was carried out on test items Interferon-alpha 1 (sample A), HYBRID 2 (sample B) and Interferon-beta 1a (sample C). These items were tested with human immunodeficiency virus type 1 (HIV-1) LAI strain. All test-items were provided in solid from which 0.25mg/mL (sample A), 1.0mg/mL (sample B) and 0.02mg/mL (sample C) were prepared in ddH2O, aliquoted, and stored at -80C to prevent repeated freeze thaw cycles.

The HIV antiviral assay utilizes HeLa-CD4-βgal cells to evaluate inhibition of HIV-1 infection. Infection with HIV induces expression of beta-galactosidase. HeLa-CD4- βgal cells were infected with HIV LAI in the presence of different concentrations of test-items. HIV LAI virus stocks were produced by transfection of 293T cells with proviral DNA, collected after 48 hours and frozen until infection. In this project, test-items were pre-incubated with target cells for 30 minutes at 37°C before the addition of HIV LAI virus to the cells. Inhibitors were present in the cell culture medium for the duration of the infection. The extent of infection of HeLa-CD4-βgal cells was monitored after 48 hours by evaluating beta-galactosidase activity and comparing values with cells treated with vehicle alone (tissue culture medium).

A viability test was set up in parallel using the same concentrations of test-items evaluated in the antiviral assay. Viability assays were used to determine test-item-induced cytotoxicity effects in the absence of virus. Cell viability was determined by the XTT method. The viability assay was conducted for the same period of time evaluated in the antiviral assay.

Nine concentrations of the test-items were evaluated in duplicates. Ten-fold serial dilutions starting at 10µg/mL were evaluated. When possible, IC50 and CC50 values for the inhibitors were determined for each assay using *GraphPad Prism* software.

### Antiviral activities of test-items A (Interferon-alpha 14), B (HYBRID 2) and C (Interferon-beta 1a)

All test-items, *A, B, and C* displayed antiviral activity against HIV-LAI. IC50 values generated with *GraphPad* software were 0.04ng/mL, 0.03ng/mL and 113.1ng/mL, for A, *B, and C,* respectively (*Table 1*). Test-items A and B did not completely block HIV infectivity, and about 25% of the infection signal was still observed even at the highest concentrations tested (10µg/mL). The reasons for this are unknown, but it is possible that a fraction of the culture is not sensitive to the activity of test-items *A* and *B.* A control inhibitor evaluated in parallel, T-20 (*enfuvirtide*), completely blocked HIV infection with minimal loss of cell viability. T-20 is a fusion inhibitor peptide that blocks HIV entry.

It is important to note that the inhibition profiles observed for test-items *A* and *B* may lead to IC50 values different from the concentrations that result in 50% inhibition of HIV infectivity. Values generated by *GraphPad Prism* were lower than the concentrations achieving 50% inhibition.

Treatments of HeLa-CD4-beta-gal cells with test-item C resulted in a small reduction of cell viability (~25%) at the highest concentration tested (10µg/mL) (*Figure 6**, page 11*). A dosedependent trend of cytotoxicity was also observed with test-item *A*. The selectivity indices for anti-HIV activity were significantly higher for test-items *A* and *B, as compared to item* C.

### Control inhibitors and quality controls

Quality controls for the infectivity assay were performed on every plate to determine: i) signal to background (S/B) values; ii) inhibition by a known inhibitor, and iii) variation of the assay, as measured by the coefficient of variation (C.V.) of all replicate test-item data points. All controls worked as anticipated for each assay. A known antiviral for HIV (T20) blocked infection over 99% at 0.5µM, and the viability controls (emetine and 10% DMSO) used in XTT cytotoxicity assay inhibited cell viability more than 95%. Overall variation in the infection assays was 5.7%, and overall variation in the viability assays was 6.2%. The signal-to-background (S/B) in the infection assay was 255-fold and in the viability assay was 7.8-fold.

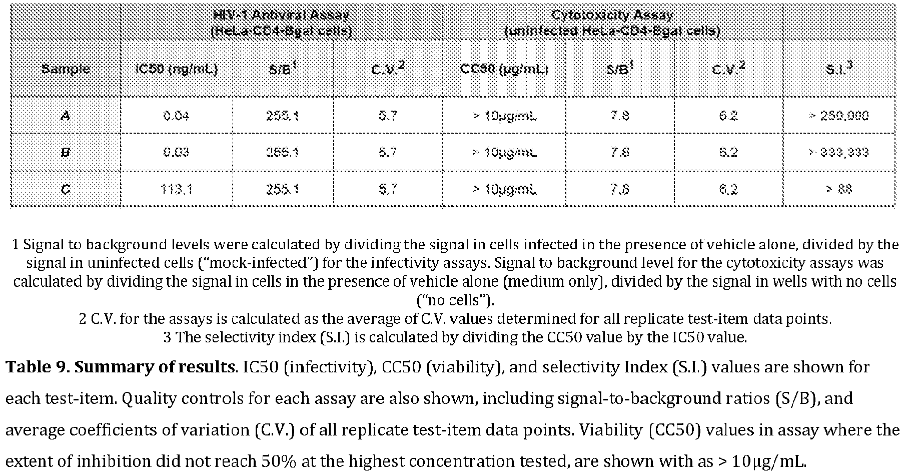

### Experimental Procedures - Antiviral assay with β-galactosidase readout

### HIV-1 Infectivity assay

Infectivity assays were performed by challenging HeLa-CD4-βgal cells with HIV-1 LAI in the presence or absence of test-items. HeLa-CD4-βgal cells express beta-galactosidase under the control of the HIV-1 LTR promoter. Upon infection with HIV and expression of the viral Tat protein beta-galactosidase expression is induced. HeLa-CD4-βgal cells were seeded at 12,000 cells per well in a white TC-treated 96-well flat bottom plate and maintained in DMEM supplemented with 10% fetal bovine serum (FBS), hereby called DMEM10. Test-items were diluted 10-fold in U-bottom plates using DMEM10. Test material dilutions were prepared at 1.25X the final concentration. Cells were incubated with the 1.25X-diluted testmaterial (80µL per well) for 30 minutes at 37°C in 5% CO2. Following the test material pre-incubation, HIV-LAI virus prepared in DMEM10 was added to the cells (20µL per well) and plates were incubated at 37°C in a humidified incubator with 5% CO2 for 48 hours. Each well contained 100µL final volume. The volume of virus used in the assay was previously determined to produce a signal in the linear range inhibited by TAF (*tenofovir alafenamide*) and T20 (*enfuvirtide*), known HIV-1 inhibitors. Infections were performed in duplicate reactions. After two days of infection, cells were monitored for beta-galactosidase activity using the Galacton-Star^{®} kit (*ThermoFisher*). The Galacto-Star test is a sensitive chemiluminescent assay that detects beta-galactosidase in mammalian cell lysates. Luminescence was measured with a 1-second readout.

Nine data points from duplicate of serial ten-fold dilutions of the test-items were evaluated ranging from 0.0001 ng/mL to 10µg/mL, were evaluated. Controls included cells incubated with no virus ("mock-infected"), infected and incubated with vehicle alone (DMEM10), or infected in the presence of *T20* (known inhibitor of HIV infection) at 0.5µM.

### QC HIV-1 infectivity assay

The average S/B in the assay was 255 (background determined with "mock-infected" cells), whereas the average variation for all data points was 5.7% (C.V. values) and 5.8% (C.V. values for all wells displaying greater than 50% infection). Background levels observed in mock-infected cells in the absence of virus were subtracted from all samples. The control antiviral used in this assay (T-20 at 0.5µM) blocked infection near 100%.

### Experimental Procedure - Cytotoxicity assays

### Viability assay (XTT) to assess test-item-induced cytotoxicity

Uninfected cells were incubated with test-items or control viability inhibitor dilutions using the same test-item concentrations as those used in the infectivity assay. The incubation temperature and duration of the incubation period mirrored the conditions of the corresponding infectivity assay. Cell viability was evaluated with the XTT method. The tetrazolium salt (XTT) is cleaved to an orange formazan dye throughout a reaction that occurs only in viable cells with active mitochondria. The formazan dye is directly quantified using a scanning multi-well spectrophotometer. Background levels obtained from wells with no cells were subtracted from all data-points. The extent of viability was monitored by measuring absorbance at 490 nm.

### QC and analysis of cytotoxicity data

The average signal obtained in wells with no cells was subtracted from all samples. Readout values were given as a percentage of the average signal observed in uninfected cells treated with vehicle alone (medium only). The signal-to-background (S/B) obtained was 7.8. *Emetine* (10µM) and 10% DMSO were used as cytotoxic controls in the viability assays and inhibited cell viability greater than 95%.

### Results - Human immunodeficiency virus type 1 (HIV-1) antiviral activity

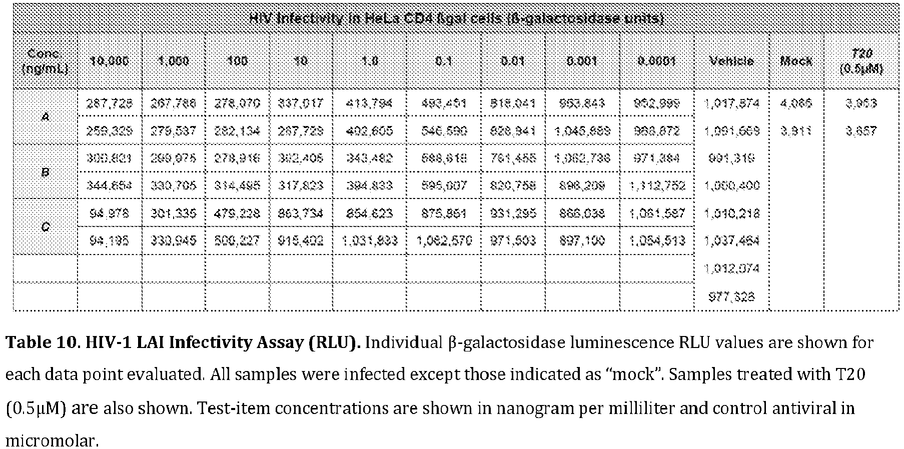

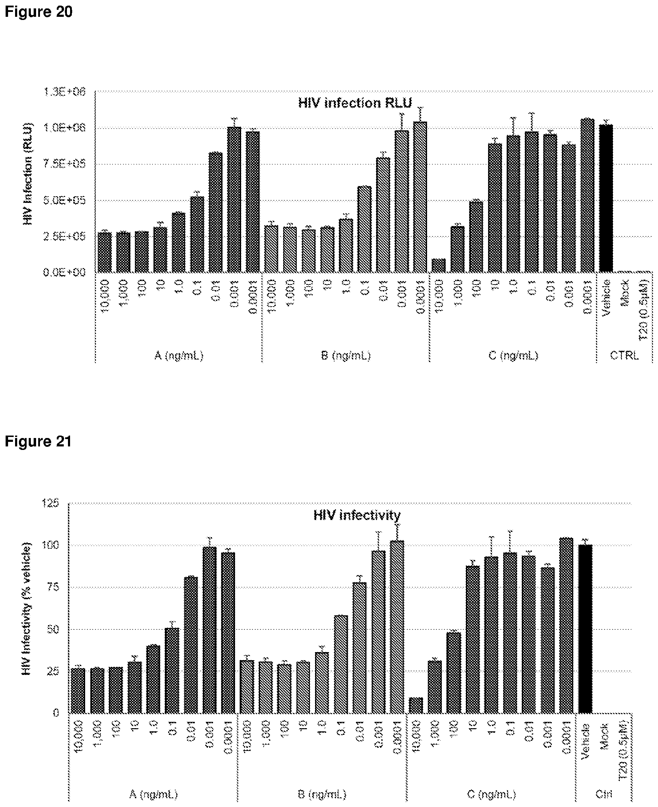

**Figure 20** **shows HIV-1 LAI infectivity by test-items Interferon-alpha 1 (sample A), HYBRID 2 (sample B) and Interferon-beta 1a (sample C) and control inhibitors (RLU).** Data is shown as relative luminescence units (RLU) values in wells containing HeLa-CD4-βgal cells infected in the presence of either vehicle alone or varying concentrations of test-items (average of duplicates with standard deviation). Uninfected cells are shown as "Mock". *T20* is included as the antiviral control (average of duplicate data points at 0.5µM).

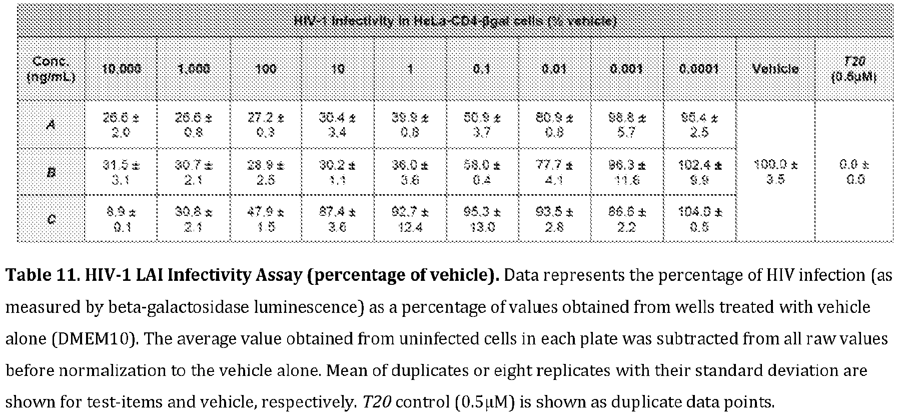

**Figure 21** **shows HIV-1 LAI Infectivity Assay (percentage values).** Results show the extent of HIV-1 LAI infection, as determined by a beta-galactosidase assay (luminescence readout) at 48 hours. Data is normalized to the activity observed in cells in the absence of test-item (vehicle alone). Test-item and control antiviral results show the average of duplicate data points with the standard deviation (s.d.), whereas the vehicle show the average and s.d. of eight replicates.

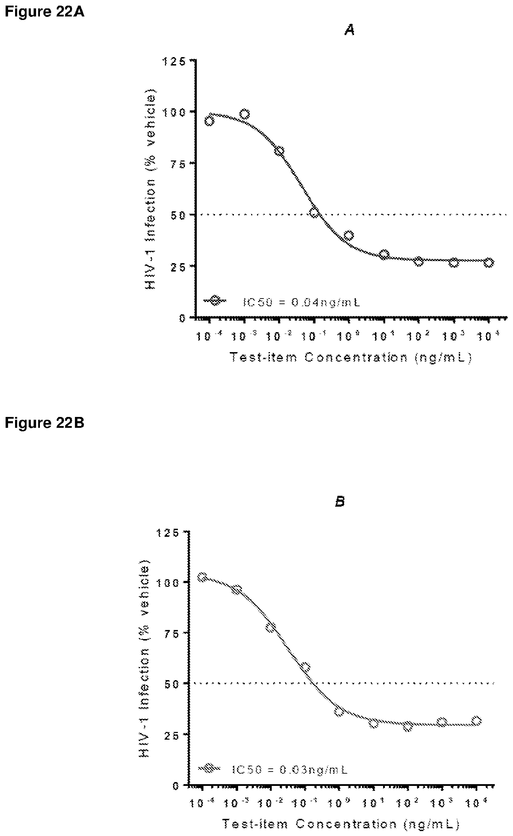

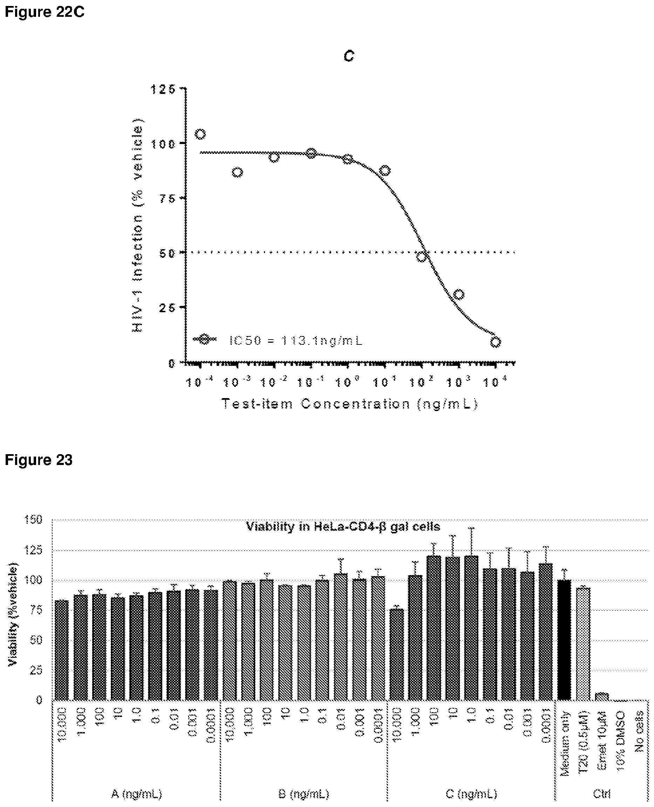

**Figure 22A** **(Interferon-alpha 14),** **Figure 22B** **(HYBRID 2) and** **Figure 22C** **(Interferon-beta 1a) show Determination of IC50 values for HIV-1 infectivity.** Values indicate the percentage of HIV infectivity compared to samples incubated with vehicle alone. Results show the average of duplicate data points with the standard deviation (s.d.). Data was adjusted to a sigmoid function and IC50 values were calculated using *GraphPad Prism* software fitting a dose-response curve with a variable slope (four parameters). IC50 values are summarized in *Table 10.*

### Results - Cytotoxicity for human immunodeficiency virus type 1 (HIV-1) assay

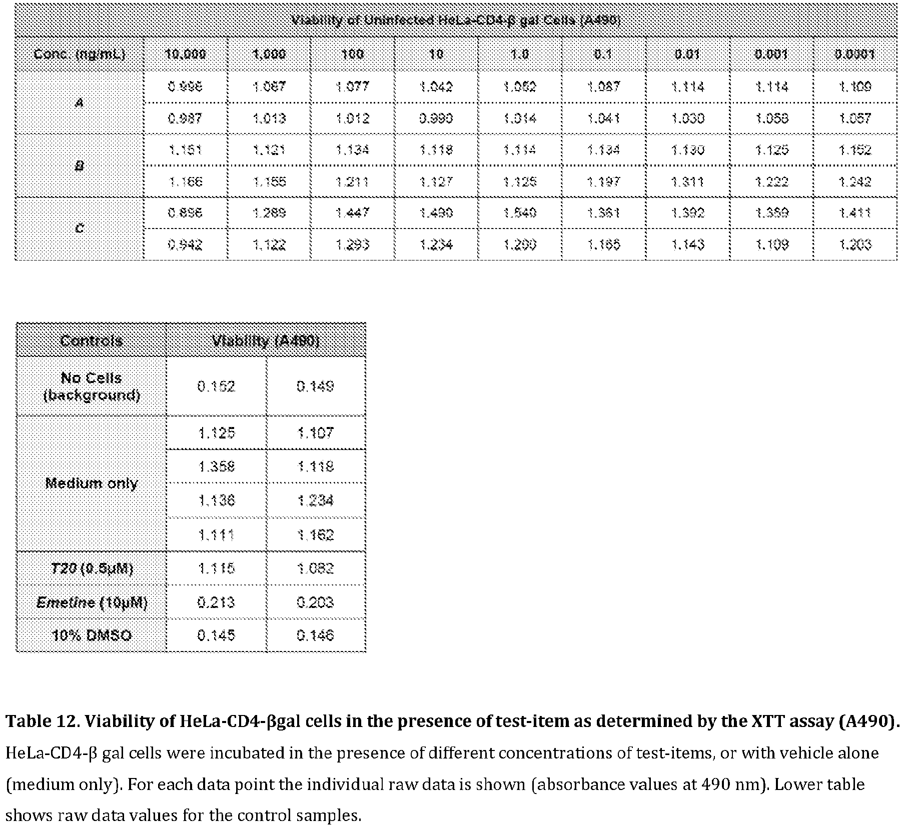

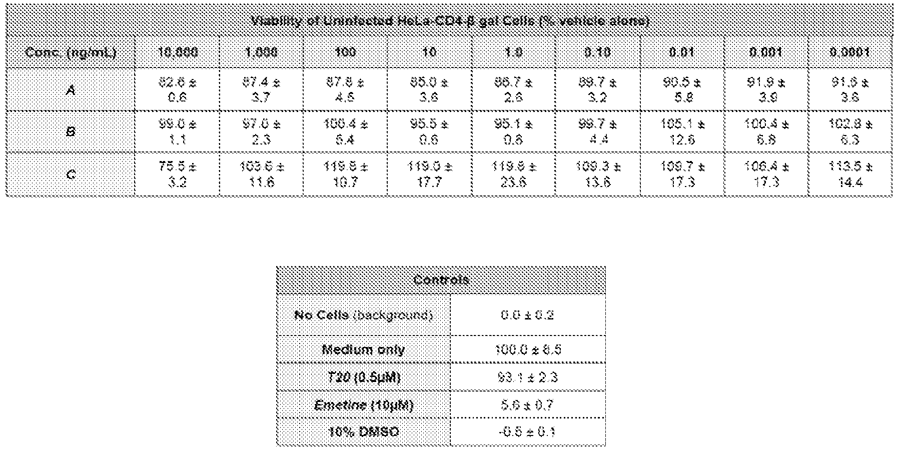

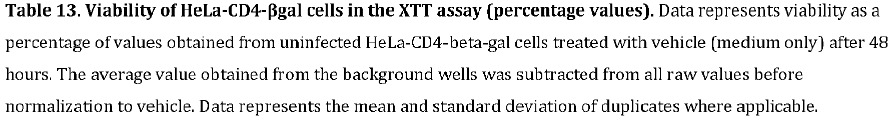

**Figure 23** **shows Viability Assay with HeLa-CD4-βgal cells (percentage values).** Results show the extent of compound-induced cytotoxicity in HeLa-CD4-beta-gal cells incubated for 48 hours, as determined by an XTT readout for viability (absorbance 490 nm readout). Data is normalized to the values observed in cells in the absence of test-item vehicle alone (medium only). Results show the average of duplicate data points with the standard deviation (s.d) for test-items and the controls *T20 (0.5*µM), emetine 10µM and 10% DMSO.

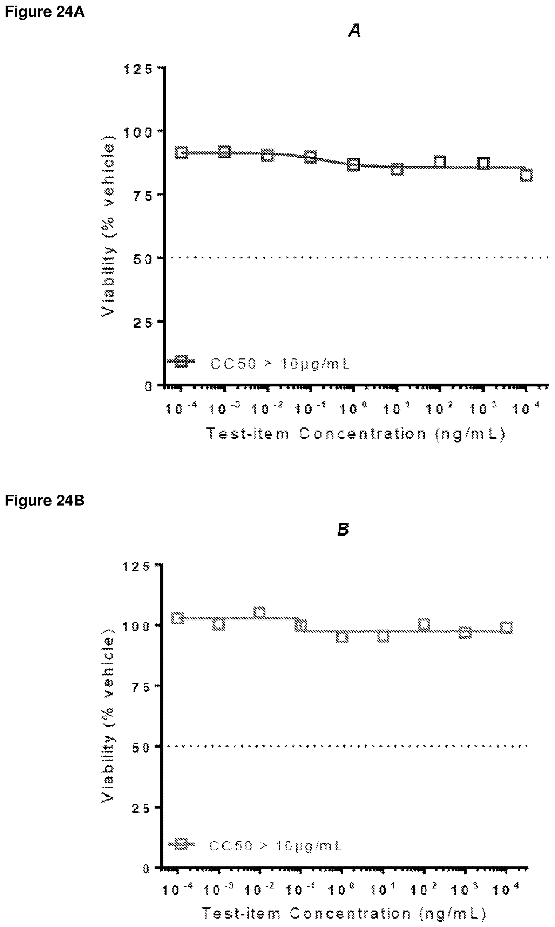

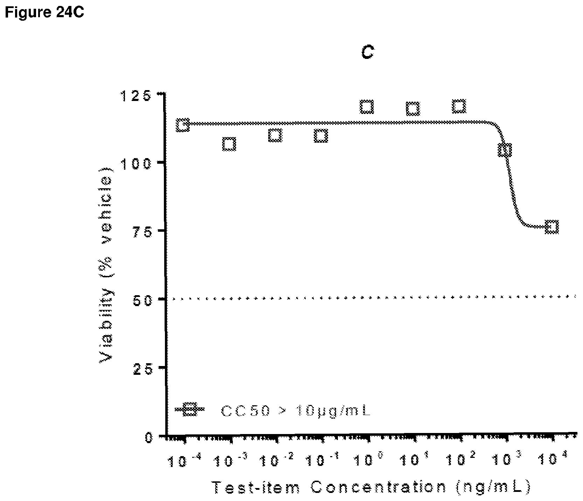

**Figure 24** **shows Determination of CC50 values for sample A (****Figure 24A****), sample B (****Figure 24B****) and sample C (****Figure 24C****) in HeLa-CD4-βgal cells (percentage values).** Results show the extent of test-item-induced cytotoxicity in HeLa-CD4-beta-gal cells incubated for 48 hours, as determined by an XTT readout for viability (absorbance 490 nm readout). Values indicate the percent viability estimated as percentage of that observed in samples incubated with vehicle alone (medium only). Results show the average of duplicate data points. Data was adjusted to a sigmoid function and CC50 values were calculated using *GraphPad Prism* software fitting a dose-response curve with a variable slope (four parameters). When viability did not reach 50%, the CC50 value reported was greater than the highest concentration tested, 10µg/mL. CC50 values are indicated in *Table 10.*

### Experiment 6: The effect of Interferon Alpha Hybrid on Herpes simplex virus type 1 (HSV1)

Experiments were carried out to determine whether treatment of primary human keratinocytes with hybrid interferon reduces HSV1 infection. The interferons used were Hybrid 1, Hybrid 2, Interferon alpha-14, Interferon alpha-2a and Interferon beta-1a.

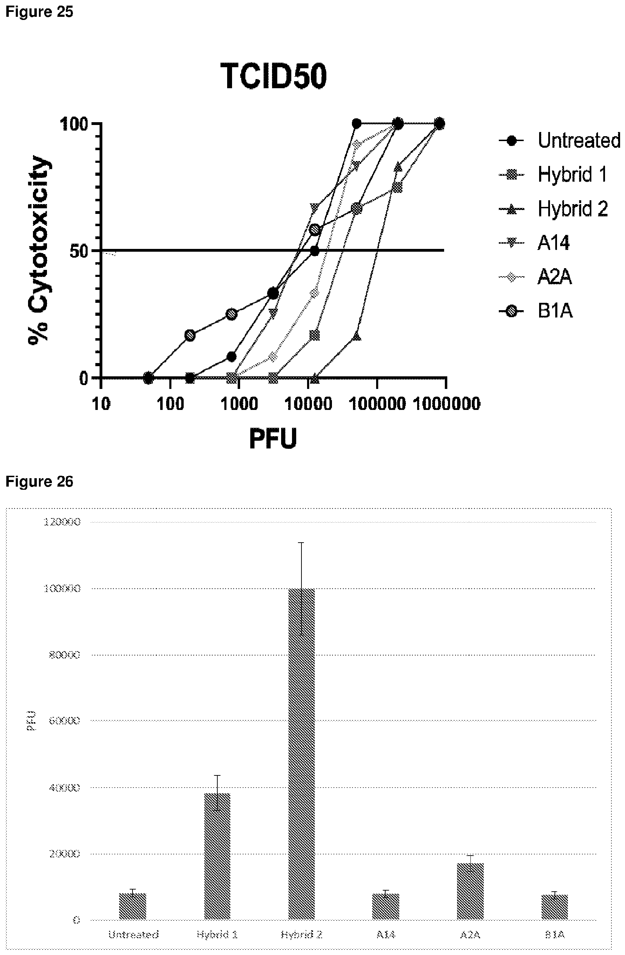

**Figure 25** shows the Tissue Culture Infectious Dose (TCID) of Herpes Simplex 1 virus in VERO cells when treated with Hybrid 1, Hybrid 2, Interferon alpha-14, Interferon alpha-2a and Interferon beta-1a.

**Figure 26** shows the median TCID converted into plaque-forming units (PFU).

### Materials and Methods

1. Propagation of Vero cells from frozen stocks (1).
2. Propagation of primary adult human epidermal keratinocytes (HEKa cells) from frozen stocks
3. Infection of HEKa cells with HSV1
4. Administration of interferons
5. Plaque assay of cell supernatant (2).
6. Processing of infected HEKa cells for FACS, ICC, RT-qPCR
7. Freezing down Vero cells
8. Freezing down keratinocytes

### Propagation of Vero cells from frozen stocks

### Materials:

African Green Monkey Kidney Vero cells (Merck 84113001-1VL; ATCC CCL-81), Cell culture incubator (37°C, 95% air, 5% CO₂), 1x PBS (no calcium or magnesium) (Sigma D8537), Trypsin-EDTA (0.25% trypsin, 0.02% EDTA) (Sigma T4049), Cell culture media (high glucose, DMEM, 10% FBS, 2mM glutamine, 1% Pen/Strep), 25 cm² and 75 cm² vented cell culture flasks, Water bath (37°C), Centrifuge and 15 ml centrifuge tube

### Method:

### Seeding cells from frozen stocks

Removed cryovial from liquid nitrogen or ultra-low temperature freezer and thaw quickly in 37°C water bath. Once thawed, transfer immediately to 15 ml centrifuge tube containing 10 ml pre-warmed cell culture media. Centrifuge 200 g for 5 mins at room temperature (RT). Discard supernatant. Resuspend in 5 ml cell culture media and place in 25 cm² vented cell culture flask. Change media every 3-4 days until cells become > 90% confluent. Vero cells recover slowly after freezing and could take > 1 week to become confluent. May need to be passaged 2-3 times before cells reach normal growth rate.

### Passaging cells (volumesfor 25 cm² flask, x2 for 7.5 cm² flask)

Remove media. Wash cells with 5 ml 1x PBS. Add 2.5 ml trypsin-EDTA, incubate at 37°C for 2-3 mins or until cells start to streak as they detach. Tap/shake flask gently to aid detachment. Can use less trypsin and incubate longer if necessary. Add 2.5 ml media and pipette to wash cells and breakup clumps. Transfer to 15 ml centrifuge tube and centrifuge at 200 g for 5 mins at RT. Discard supernatant and resuspend in 10 ml media. Can split between 1:5 and 1:10 for maintenance. Seed 2 × 10^5 cells per well in 12-well plate for plaque assay.

### Plaque assay of cell supernatants

### Materials:

Vero cells, Vero cell media, Supernatant, 1.5 % methylcellulose (Sigma M6385-100G) in 1X PBS, 1 % crystal violet (Sigma C0775-25G) in 1:1 ratio MeOH:H₂O

### Method:

### Day -2: Prepare 1.5 % methylcellulose

Add 1.5 g methylcellulose to 100 ml 1X PBS. Autoclave for 45 mins on liquid cycle. Allow cool. Add 350 ml Vero cell media. Stir overnight at 4°C.

### Day -1: Seed Vero cells

Label one 12-well plate per condition. Split cells as described previously. Seed 2 × 10^5 Vero cells per well of a 12-well plate. Allow settle for 15-30 mins before returning to the incubator.

### Day 0: Infection of Vero cells

Check Vero cells are confluent. Prepare a series dilution of the supernatants in cell culture media. Dilutions should range between 10^-2 and 10^-7. Need 200 µl of each dilution per well, performed in duplicate. Remove Vero cell media. Add 200 µl of the appropriate dilution to each well. Place in incubator. Shake plates every 15 mins for 1 hr. Aspirate virus and overlay with 1.5 ml methylcellulose. Incubate 3-5 days or until plaques are visible. Remove overlay and add 2 ml crystal violet. Incubate 10 - 20 mins at RT. Aspirate stain and rinse gently with tap water. Allow dry. (No. plaques)/(volume inoculum (ml))*(dilution) = pfu / ml.

### Freezing down Vero cells

### Materials:

1 75 cm² flask confluent Vero cells, Freezing media (High glucose DMEM, 20% FBS), Dimethyl sulfoxide (DMSO), DPBS no calcium magnesium, Trypsin-EDTA, Cryovials, 15 ml centrifuge tubes, Cryo Cell freezing container, Isopropanol

### Method:

Add 1 ml DMSO to 9 ml Freezing Media and set aside. Label 10 cryovials with date, cell-type and passage number. Remove media from flask. Wash with 10 ml DPBS. Add 5 ml trypsin-EDTA, incubate 37°C for 2-3 mins. Add 5 ml Freezing Media, wash cells, break up clumps. Transfer cells to 15 ml centrifuge tube. Centrifuge 200 g for 5 mins. Discard supernatant and resuspend in 10 ml DMSO-Freezing Media. Add 1 ml cells to each cryovial and transfer immediately to freezing chamber containing isopropanol. Store at -80°C overnight. Transfer to liquid nitrogen for long-term storage.

Various modifications and variations to the described embodiments of the inventions will be apparent to those skilled in the art without departing from the scope of the invention. Although the invention has been described in connection with specific preferred embodiments, it should be understood that the invention should not be unduly limited to such specific embodiments. Indeed, various modifications of the described modes of carrying out the invention which are obvious to those skilled in the art are intended to be covered by the present invention.

## Claims

1. An interferon alpha subtype, HYBRID 2 for use in the treatment and/or prophylaxis of a virus or viral infection;
wherein the interferon alpha subtype, HYBRID 2, comprises or consists of the amino acid sequence of SEQ ID NO:3 or a functionally active fragment of SEQ ID NO:3; and
wherein the virus or viral infection is selected from a member of the Flaviviridae family, the Arenaviridae family, the Bunyaviridae family, the Togaviridae family, the Herpesviridae family, the Orthomyxoviridae family, the Rhabdoviridae family, the Paramyxoviridae family, the Picornaviridae family or the Retroviridae family.

2. The interferon alpha subtype for use in the treatment and/or prophylaxis of a viral infection as claimed in claim 1, wherein the virus or viral infection is selected from Chikungunya virus (CHIKV)
Coxsackievirus
Dengue virus (DENV)
Epstein-Barr virus (EBV
Hepatitis B virus (HBV)
Hepatitis C virus (HCV)
Human cytomegalovirus (HCMV)
Human immuno-deficiency virus (HIV)
Human parainfluenza virus (HPIV)
Human respiratory syncytial virus (HRSV)
Human rhinovirus (HRV)
Herpes simplex virus (HSV)
Influenza A virus (IAV)
Influenza B virus (IBV)
Lassa virus (LASV)
Measles virus (MeV)
Mumps virus (MuV)
Nipah virus (NiV)
Poliovirus (PV)
Rabies virus (RABV)
West Nile virus (WNV)
Yellow fever virus (YFV)
Zika Virus (ZIKV).

3. The interferon alpha subtype for use in the treatment and/or prophylaxis of a viral infection as claimed in claim 1,wherein the viral infection is treated by providing HYBRID 2 (SEQ ID NO:3 or a functionally active fragment thereof), by direct injection or where appropriate for viruses that cause respiratory issues, by aerosol directly into the lungs.

4. The interferon alpha subtype for use in the treatment and/or prophylaxis of a viral infection as claimed in claim 1, wherein the virus or viral infection is selected from Human Immunodeficiency Virus (HIV), Human Respiratory Syncytial Virus (HRSV) and Herpes Simplex Virus (HSV).

5. The interferon alpha subtype for use in the treatment and/or prophylaxis of a viral infection as claimed in claim 1, further comprising a therapeutically useful amount of an additional anti-viral treatment.

6. The interferon alpha subtype for use in the treatment and/or prophylaxis of a viral infection as claimed in claim 5, wherein the anti-viral treatment is ribavirin, Remdesivir, LAM-002A, dexamethasone, Avigan (favilavir) or another interferon subtype.

## Patentansprüche

1. Ein Interferon-alpha-Subtyp, HYBRID 2, zur Verwendung bei der Behandlung und/oder Prophylaxe gegen ein Virus oder eine Virusinfektion;
wobei der Interferon-alpha-Subtyp, HYBRID 2, die Aminosäuresequenz von SEQ ID NO: 3 oder ein funktionell aktives Fragment von SEQ ID NO: 3 beinhaltet oder daraus besteht; und
wobei das Virus oder die Virusinfektion ausgewählt ist aus einem Mitglied der Flaviviridae-Familie, der Arenaviridae-Familie, der Bunyaviridae-Familie, der Togaviridae-Familie, der Herpesviridae-Familie, der Orthomyxoviridae-Familie, der Rhabdoviridae-Familie, der Paramyxoviridae-Familie, der Picornaviridae-Familie oder der Retroviridae-Familie.

2. Interferon-alpha-Subtyp zur Verwendung bei der Behandlung und/oder Prophylaxe gegen eine Virusinfektion gemäß Anspruch 1, wobei das Virus oder die Virusinfektion ausgewählt ist aus
Chikungunya-Virus (CHIKV),
Coxsackie-Virus,
Dengue-Virus (DENY),
Epstein-Barr-Virus (EBV),
Hepatitis-B-Virus (HBV),
Hepatitis-C-Virus (HCV),
Humanem Zytomegalie-Virus (HCMV),
Humanem Immundefizienz-Virus (HIV),
Humanem Parainfluenza-Virus (HPIV),
Humanem Respiratorischem Synzytial-Virus (HRSV),
Humanem Rhinovirus (HRV),
Herpes-simplex-Virus (HSV),
Influenza-A-Virus (IAV),
Influenza-B-Virus (IBV),
Lassavirus (LASV),
Masernvirus (MeV),
Mumpsvirus (MuV),
Nipahvirus (NiV),
Poliovirus (PV),
Rabiesvirus (RABV),
West-Nil-Virus (WNV),
Gelbfiebervirus (YFV),
Zikavirus (ZIKV).

3. Interferon-alpha-Subtyp zur Verwendung bei der Behandlung und/oder Prophylaxe gegen eine Virusinfektion gemäß Anspruch 1, wobei die Virusinfektion durch Bereitstellen des HYBRID 2 (SEQ ID NO: 3 oder ein funktionell aktives Fragment davon) durch direkte Injektion oder, wo angebracht, für Viren, die Atemprobleme verursachen, durch Aerosol direkt in die Lungen behandelt wird.

4. Interferon-alpha-Subtyp zur Verwendung bei der Behandlung und/oder Prophylaxe gegen eine Virusinfektion gemäß Anspruch 1, wobei das Virus oder die Virusinfektion ausgewählt ist aus Humanem Immundefizienz-Virus (HIV), Humanem Respiratorischem Synzytial-Virus (HRSV) und Herpes-simplex-Virus (HSV).

5. Interferon-alpha-Subtyp zur Verwendung bei der Behandlung und/oder Prophylaxe gegen eine Virusinfektion gemäß Anspruch 1, der ferner eine therapeutisch nützliche Menge einer zusätzlichen antiviralen Behandlung beinhaltet.

6. Interferon-alpha-Subtyp zur Verwendung bei der Behandlung und/oder Prophylaxe gegen eine Virusinfektion gemäß Anspruch 5, wobei die antivirale Behandlung Ribavirin, Remdesivir, LAM-002A, Dexamethason, Avigan (Favilavir) oder ein anderer Interferon-Subtyp ist.

## Revendications

1. Un sous-type de l'interféron alpha, HYBRID 2, pour son utilisation dans le traitement et/ou la prophylaxie d'un virus ou d'une infection virale ;
où le sous-type de l'interféron alpha, HYBRID 2, comprend ou consiste en la séquence d'acides aminés de SEQ ID NO : 3 ou en un fragment fonctionnellement actif de SEQ ID NO : 3 ; et
où le virus ou l'infection virale est sélectionné(e) parmi un membre de la famille des Flaviviridae, de la famille des Arenaviridae, de la famille des Bunyaviridae, de la famille des Togaviridae, de la famille des Herpesviridae, de la famille des Orthomyxoviridae, de la famille des Rhabdoviridae, de la famille des Paramyxoviridae, de la famille des Picornaviridae ou de la famille des Retroviridae.

2. Le sous-type de l'interféron alpha pour son utilisation dans le traitement et/ou la prophylaxie d'une infection virale tel que revendiqué dans la revendication 1, où le virus ou l'infection virale est sélectionné(e) parmi
le virus chikungunya (VCHIK)
le virus coxsackie
le virus de la dengue (VDEN)
le virus d'Epstein-Barr (VEB)
le virus de l'hépatite B (VHB)
le virus de l'hépatite C (VHC)
le cytomégalovirus humain (CMVH)
le virus de l'immunodéficience humaine (VIH)
le virus para-influenza humain (VPIH)
le virus respiratoire syncytial humain (VRSH)
le rhinovirus humain (RVH)
le virus herpès simplex (VHS)
le virus influenza A (VIA)
le virus influenza B (VIB)
le virus de Lassa (LASV)
le virus de la rougeole (VR)
le virus des oreillons (VO)
le virus Nipah (NiV)
le poliovirus (PV)
le virus de la rage (VR)
le virus du Nil occidental (VNO)
le virus de la fièvre jaune (VFJ)
le virus Zika (VZ).

3. Le sous-type de l'interféron alpha pour son utilisation dans le traitement et/ou la prophylaxie d'une infection virale tel que revendiqué dans la revendication 1, l'infection virale étant traitée par apport de HYBRID 2 (SEQ ID NO : 3 ou un fragment fonctionnellement actif de celle-ci), par injection directe ou lorsque cela est approprié pour des virus qui causent des problèmes respiratoires, par aérosol directement dans les poumons.

4. Le sous-type de l'interféron alpha pour son utilisation dans le traitement et/ou la prophylaxie d'une infection virale tel que revendiqué dans la revendication 1, où le virus ou l'infection virale est sélectionné(e) parmi le virus de l'immunodéficience humaine (VIH), le virus respiratoire syncytial humain (VRSH) et le virus herpès simplex (VHS).

5. Le sous-type de l'interféron alpha pour son utilisation dans le traitement et/ou la prophylaxie d'une infection virale tel que revendiqué dans la revendication 1, comprenant en sus une quantité thérapeutiquement utile d'un traitement antiviral supplémentaire.

6. Le sous-type de l'interféron alpha pour son utilisation dans le traitement et/ou la prophylaxie d'une infection virale tel que revendiqué dans la revendication 5, où le traitement antiviral est la ribavirine, le remdesivir, LAM-002A, la dexaméthasone, Avigan (favilavir) ou un autre sous-type d'interféron.
